# EUROPEAN PATENT APPLICATION

(11) **EP 1 413 887 A1**
(43) Date of publication of application: **28.04.2004**
(21) Application number: 02292608.3
(22) Date of filing: 22.10.2002
(51) Int. Cl.: G01N 33/68, C12Q 1/48

(54) **Inhibitors of Src kinase for use in Alzheimer's disease**

(71) Applicant: Aventis Pharma S.A., 92160 Antony (FR)
(72) Inventor: Mercken, Luc, 94100 Saint Maur (FR); Zambrano, Nicola, 80020 Casavatore (NA) (IT); Russo, Tommaso, 80131 Napoli (IT)

(57) **Abstract**

The present invention relates to the identification of inhibitors of Src kinase, assays and methods useful for such identification and the use of Src inhibitors for the preparation of pharmaceuticals for the treatment of Alzheimer's disease.

## Description

The present invention relates to the identification of inhibitors of Src kinase, assays and methods useful for such identification and the use of Src inhibitors for the preparation of pharmaceuticals for the treatment of Alzheimer's disease.

Alzheimer's disease is a neurodegenerative disorder of the brain, which is accompanied at the cellular level by a massive loss of neurons in the limbic system and in the cerebral cortex. In the brain areas affected, protein deposits, so-called plaques, can be detected at the molecular level, which are an essential characteristic of Alzheimer's disease. The protein occurring most frequently in these plaques is a peptide of 40 to 42 amino acids in size, which is designated as Aβ-peptide. This peptide is a cleavage product of a larger protein of 695 to 770 amino acids, the so-called Amyloid Precursor Protein (APP).

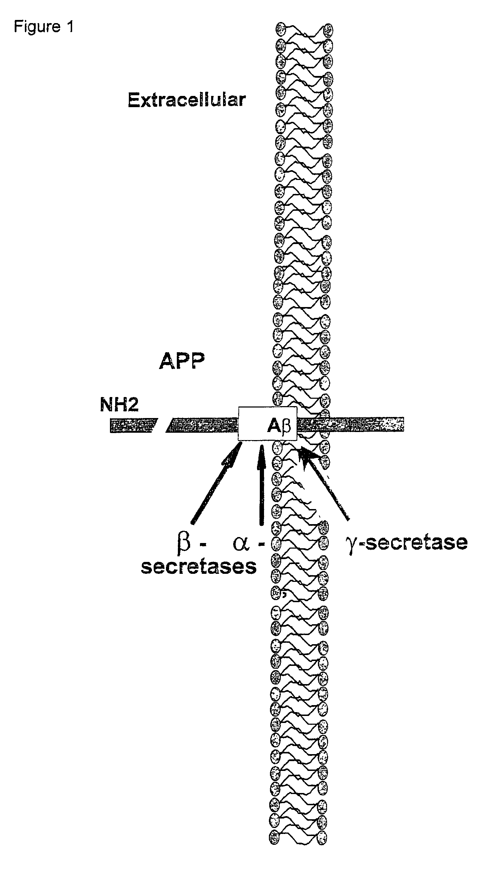

APP is an integral transmembrane protein, which firstly traverses the lipid bilayer. By far the largest part of the protein is extracellular, while the shorter C-terminal domain is directed into the cytosol (Figure 1). APP is the substrate of three different proteases: α-secretase, β-secretase and γ-secretase. Within APP, about two thirds of the Aβ-peptide originates from the extracellular domain and about one third from the transmembrane domain.

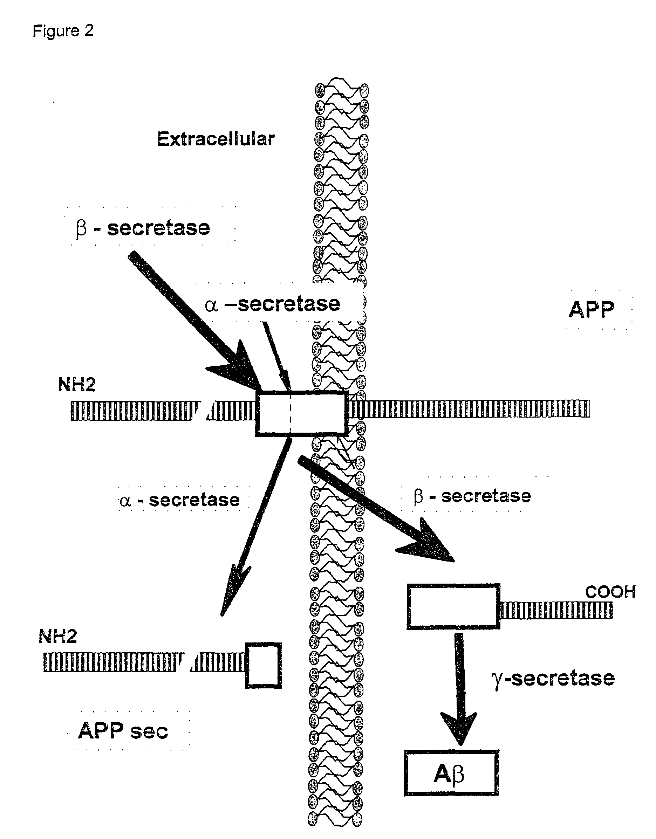

Beside the membrane-linked APP, a secreted form of APP can be detected which consists of the large ectodomain of APP and is designated as APP_{sec} ("secreted APP"). APP_{sec} is formed from APP by proteolytic cleavage, which is effected by the α-secretase (Figure 2). This proteolytic cleavage takes place within the amino acid sequence of the Aβ-peptide (after amino acid residue 16 of the Aβ-peptide). Proteolysis of APP by the α-secretase thus excludes the formation of the Aβ-peptide.

The Aβ-peptide can thus only be formed from APP in an alternative processing route. It is postulated that two proteases are involved in this processing, one protease, which is designated as β-secretase, cleaving at the N-terminus of the Aβ-peptide in the APP and the second protease, which is designated as γ-secretase, releasing the C-terminus of the Aβ-peptide (Kang, J. et al., (1987) Nature, 325, 733 (Figure 2).

There are many indications that the Aβ-peptide is a crucial factor in the occurrence of Alzheimer's disease. Inter alia, neurotoxicity of Aβ-fibrils in cell culture is postulated (Yankner, B.A. et al., (1990) Proc Natl Acad Sci USA, 87, 9020). In patients with Down's syndrome, in which APP occurs in an additional copy, the neuropathology characteristic of Alzheimer's disease also occurs even at an age of 30 years. Here, it is assumed that the overexpression of APP follows an increased conversion into the Aβ-peptide (Rumble, B. et al., (1989), N. Engl. J. Med., 320, 1446).

Furthermore the strongest indication of the central role of the Aβ-peptide in Alzheimer's disease are the familial forms of the disease. Here, mutations are found in the APP gene around the area of the secretase cleavage sites or in two further AD-associated genes (presenilins), which in cell culture lead to a significant increase in Aβ production (Scheuner, D. et al., (1996), Nature Medicine, 2, 864).

There are a number of indications that APP is first cleaved by the β-secretase, to serve thereafter as a substrate for γ-secretase (Maruyama, K. Y. et al., (1994) Biochem. Biophys Res Commun, 202, 1517; Estus, S. et al., (1992), Science, 255, 726 ). The γ-secretase therefore has a crucial role in the formation of the Aβ-peptide. A demonstration of the activity of the γ-secretase which is customarily used is the detection of the Aβ-peptide, which, however, frequently turns out to be difficult.

An important reason for this is that only a small part of APP is converted into the Aβ-peptide (Simons M, et al., Neurosci (1996) 1;16(3):899-908). Moreover, the Aβ-peptide is a small breakage fragment of about 4 kDa and, on account of its hydrophobic character, has a great tendency to self-aggregation so that it easily precipitates under physiological conditions (Hilbich, C. et al., (1991) J. Mol. Biol., 218, 149).

The short, 47 amino acid-long C-terminal tail of APP is exposed to the cytosol and is the site of interaction for molecular adaptors containing PTB domains, named Fe65, X11, m-dab and Jip1. Since the binding of these proteins is dependent on the YENPTY sequence on APP, it is expected that their interactions are not simultaneous (T. Russo et al. (1998) FEBS Lett. 434, 1-7; N. Zambrano et al. (1997) J. Biol. Chem. 272, 6399-6405). Indeed, it has been shown, in cultured cells, that Fe65 and X11 proteins have opposite effects on the proteolytic processing of APP (Sabo S. et al. (1999) J. Biol. Chem. 274, 7952-7957; Borg J.P. et al. (1998) J. Biol. Chem. 273, 14761-14766). A possible explanation for these findings may reside in the recruitment, by either Fe65 or X11, of APP in different macromolecular complexes, depending on the interaction of different sets of proteins with the other protein-protein interaction domains of the two adaptors.

The APP cytodomain contains various serine and threonine residues, which are phosphorylated *in vitro* by different kinase activities. Thr668 is a main site of phosphorylation *in vivo*, being phosphorylated by neuronal cyclin-dependent kinases, Cdk5 in neurons (lijima, K. et al. (2000) J. Neurochem. 75, 1085-1091), cdc2 kinase in dividing cells (Suzuki, T. et al. (1994) EMBO J. 13, 1114-1122 ; Oishi, M. et al. (1997) Mol. Med. 3, 111-123), and glycogen synthase kinase 3b (GSK3b) and stress-activated protein kinase 1b (SAP kinase1b) *in vitro* (Aplin, E. E. et al. (1996) J. Neurochem. 67, 699-707 ; Standen, C. L. et al. (2001) J. Neurochem. 76, 316-320). It has been shown that phosphorylation of APP regulates neurite extension in differentiating PC12 cells (Ando, K. et al. (1999) J. Neurosci. 19, 4421-4427), while, at the molecular level, APP phosphorylation may regulate the binding to the PTB domain-containing adaptors and its processing (Ando K. et al. (2001) J Biol. Chem., 276, 40353-40361).

Previously it was demonstrated that APP is phosphorylated at tyrosine 682 in cells expressing an active form of the Abl non receptor tyrosine kinase (Zambrano N. et al. (2001) J Biol. Chem. 276, 19787-92); active Abl is recruited in proximity to APP by Fe65, which may bind simultaneously these two proteins through its WW domain (Abl) and PTB2 domain (APP). Phosphorylation of Tyr682 of APP generates a docking site for the SH2 domain of Abl and, in fact stable complexes between APP and Abl are formed.
In order to test the hypothesis that tyrosine phosphorylation regulates pp60c-src biological activity, Kmiecik and Shalloway (1987) Cell 49, 65-73 described the construction and study of pp60c-src mutants in which Tyr 527 and Tyr 416 were separately or coordinately altered to phenylalanine. Tyr---- Phe 527 mutation strongly activated pp60c-src transforming and kinase activities, whereas the additional introduction of a Tyr----Phe 416 mutation suppressed these activities. Tyr----Phe 416 mutation of normal pp60c-src eliminated its partial transforming activity, which suggests that transient or otherwise restricted phosphorylation of Tyr 416 is important for pp60c-src function even though stable phosphorylation is not observed in vivo. Normally, pp60c-src is phosphorylated in vivo at tyrosine 527, a residue not present in pp60v-src (its transforming homolog), and not at tyrosine 416, its site of in vitro autophosphorylation.

Williamson et al. (2002) J. Neurochem. 22, 10-20) described the rapid phosphorylation of neuronal proteins including Tau and Focal adhesion kinase in response to amyloid-β peptide exposure and an involvement of Src family protein kinases.

Slack, B. E. and Berse; B. (1998) described in Society for Neuroscience Abstracts, Vol. 24, No. 1-2, pp. 208 (Conference/Meeting Information: 28^{th} Annual Meeting of the Society for Neuroscience, Part 1, Los Angeles, California, USA, Nov. 7-12, 1998 Society for Neuroscience, ISSN: 0190-5295) a role for tyrosine kinases in the stimulation of APP release by action of muscarinic m3 acetylcholine receptors. They described a role for Src tyrosine kinase in the regulation of APP_{sec} release by muscarinic receptors. They demonstrated that an increase in the active form of Src leads to a decrease in secAPP.

The present invention provides methods of identifying therapeutic agents for the treatment of Alzheimer's disease.

One embodiment of the invention provides methods for identifying inhibitors of Src activity, whereby the methods comprise the steps
a) providing a Src protein (i.e. a Src encoding DNA under control of an expression element) and
b) determining if a compound inhibits the activity of Src.

The Src protein could be any mammalian Src protein, preferably human Src (SEQ ID NO. 1 (isoform 1) and SEQ ID NO. 2 (isoform 2)) or rodent Src (SEQ ID NO. 3). The protein could be obtained by expressing human Src or rodent Src cDNA, by using e. g. sequences SEQ ID NO. 4 or SEQ ID NO. 5. In addition, one of the sequences deposited under Genbank Accession No. M17031 or BC011566 can be used.

For the method preferably a mammalian cell or cell line is used in which Src is expressed, either naturally or because the cell / cell line is genetically engineered. In a particular embodiment primary cultures of neurones are used.

Another embodiment of the invention relates to a method of identifying compounds, which inhibit Src expression. The method comprises the steps
a) providing a sequence which regulates Src expression (i.e. a Src promotor sequence) and
b) determining if a compound inhibits the expression of Src protein.

The sequence which regulates Src expression is preferably linked to a reporter gene or a reporter gene construct. Such reporter gene can easily be used to determine, if a compound inhibits the expression of Src. Another possibility is the use of the Src gene. The region of chromosome 20 including the Src gene is deposited under Genbank Accession no AL133293.

An house-keeping promoter, which can be used as a sequence which regulates Src expression, has been described, as well as an alternative promoter regulated by the Hepatic Nuclear Factor-1a (Bonham et al. (2000) J. Biol. Chem. 275, 37604) (Genbank accession number AF272982).

Active compounds, this means compounds, which inhibit Src expression or which inhibit Src protein activity, can be used as pharmaceuticals. Such compounds can be used for the preparation of a pharmaceutical for the treatment or prevention of Alzheimer's disease.
Another embodiment of the invention relates to the use of compounds identified by one of the forgoing methods for preparing a pharmaceutical for the treatment of Alzheimer's disease.

The invention further relates to a method of preparing a pharmaceutical for treatment of Alzheimer's disease comprising the steps
a) identification of a therapeutic compound by the use of one of the methods described above;
b) optimisation of the identified compound; and
c) formulation of the optimised compound.

Another embodiment of the invention relates to the use of Src inhibitors known in the art for the preparation of a pharmaceutical for the treatment or the prevention of Alzheimer's disease. The invention relates to the use of PP2 (4-amino-5-(4-chlorophenyl)-7-(t-butyl)pyrazolo[3,4-d]pyrimidine) as pharmaceutical. The invention further relates to the use of PP2 for the preparation of a pharmaceutical for the treatment of Alzheime'rs disease.

Another embodiment of the invention refers to the use of PP2 for the preparation of a pharmaceutical for the treatment of Alzheimer's disease.

The new results demonstrate, that APP is phosphorylated not only by Abl. In fact Src kinase is also responsible for APP tyrosine phosphorylation. It is expected that tyrosine phosphorylation of APP alters the binding properties of its cytodomain, and then the processing of APP could be consequently modified. If so, this will prove useful the use of currently available tyrosine kinase inhibitors, and to design new ones for pharmacological modulation of the release of APP processing products in cellular and animal models of Alzheimer's disease.
Taken together, the results from the following examples suggest that exogenous Src activity is responsible for activation of a pathway resulting in increased Aβ secretion. This also suggests that PP2 could be used as a pharmacological agent able to reduce Aβ secretion in cellular and animal systems resembling AD status.

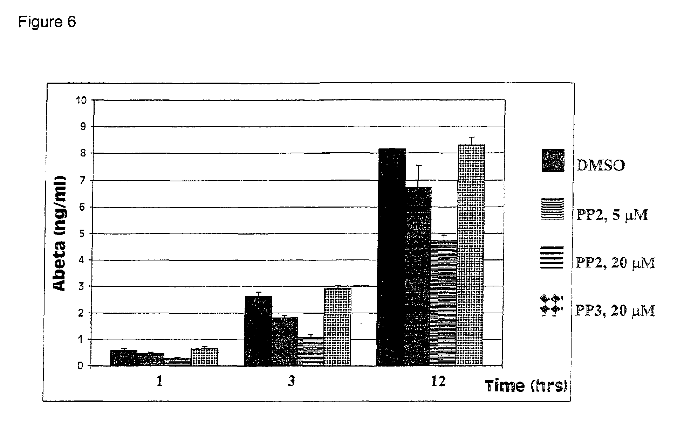

Therefore, a line of CHO cells stably overexpressing APP751 form was used, which secretes discrete levels of Aβ. If secretion involves endogenous Src activity, PP2 should be able to inhibit production of Aβ also in this cellular system. This is indeed the case since, as shown in Figure 6, PP2 reduces in a dose-dependent manner the amount of Aβ at the three time points tested (1, 3 and 12 hours), while PP3, used at the highest concentration, has no effect.
These experiments clearly outline the relevance of Src activation in Aβ secretion and constitute the basis to try pharmacological applications for tyrosine kinase inhibitors in the treatment of AD. Screening of such inhibitors and their optimization will lead to the development of potential, innovative therapeutic agents for treatment of Alzheimer's disease.

### Examples

### Example 1

### Cell culture, transfections and treatments

Human embryonic kidney cells (HEK293) were cultured in Dulbecco's modified minimal medium supplemented with 10% fetal calf serum at 37 °C in a 5% CO₂ atmosphere. For transfection, cells were grown for 16 hours in antibiotic-free medium, and transfected with Lipofectamine 2000 (invitrogen) according to manufacturer's instructions. The total amount of DNA was maintained constant by addition of empty vector DNA. 48 hours after transfection, culture medium were recovered, while cells were harvested in ice-cold phosphate buffer saline (PBS) and gently lysed in lysis buffer (50 mM Tris/HCl, pH 7.5, 150 mM NaCl, 0.5% Triton X-100, 10% glycerol, 50 mM NaF, 1mM Na vanadate), in the presence of a protease inhibitor cocktail (Complete, EDTA-free, Roche). The extracts were clarified by centrifugation at 16,000 x g at 4 °C, and the protein concentration determined by the Bio-Rad protein assay according to manufacturer's instructions. For metabolic labelling, 36 hours after transfection, cells were incubated for 30 minutes in medium without methionine and cysteine, then the medium was replaced with fresh medium containing 80 µCi/ml of ³⁵S-methionine/cysteine mixture (Promix, 1000 Ci/mmol, Amersham Pharmacia Biotech). Pulse was for 30 minutes, then the radioactive medium was replaced with complete medium for 90 minutes. CHO cells expressing wild-type APP751 have been described (N. Zambrano et al. (1997) J. Biol. Chem. 272, 6399-6405).
Immuno-precipitations were performed either on culture medium, or on cellular lysates with 1 µg/sample of the 6E10 monoclonal antibody (Signet). Radioactive samples were resolved on 10% SDS-PAGE and quantitated with a Storm 840 phosphorimager (Amersham Pharmacia Biotech).
PP2 and PP3 (Hanke, J.H. et al. (1996) J. Biol. Chem. 271, 695-701) were purchased from Calbiochem and dissolved in DMSO; treatments were performed on transfected cells for 48 hours with vehicle alone, or with 1, 5 and 20 µM PP2 or PP3. Concentration of DMSO was the same in all samples. Treatments on CHO/APP751 cells was made with DMSO, or PP2 (5 and 20 µM), or PP3 (20 µM).

### Example 2

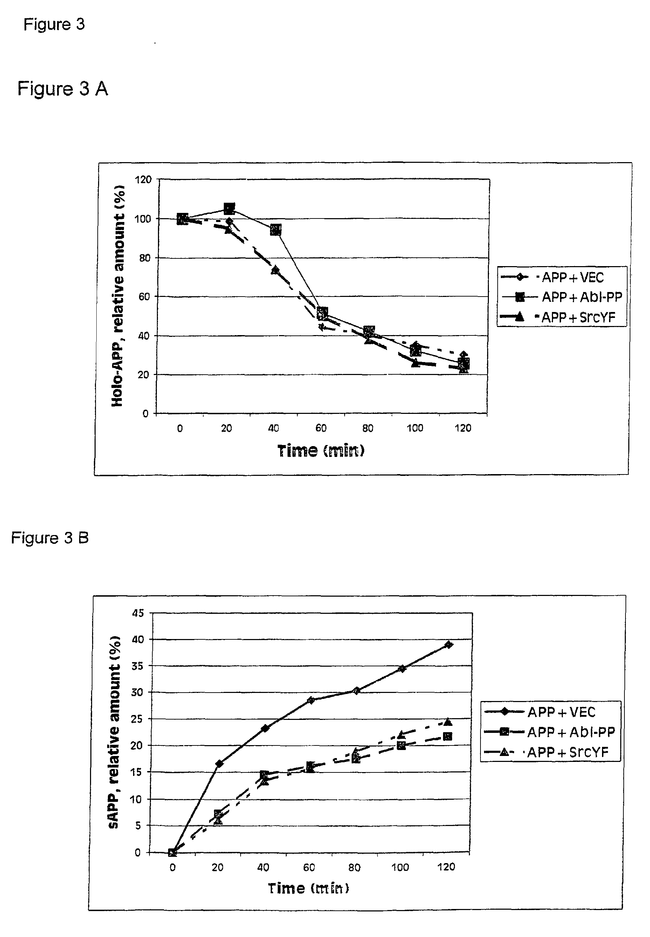

HEK293 cells were transfected with APP695 expression vectors. This cellular system was used because these cells are transfected to high efficiency, and because they express the whole set of processing activities required for APP maturation. In fact, upon transfection of human APP695 expression vectors, both α-secretase product (APP_{sec}) and β- γ-secretase products (Aβ) accumulate in the culture medium. HEK293 cells were co-transfected with APP and either empty vector, or active Abl (Abl-PP), or active Src (SrcYF) kinases, and the secretion of APP_{sec} and Aβ were evaluated. Transfected cells have been pulse-labelled with ³⁵S-Met/Cys mix for 30', and chased with cold amino acids for 90'. Both medium and protein extracts have been used in immunoprecipitation experiments for quantitative dosage of radiolabeled APP_{sec} and holo-APP, respectively. While the stability of holo-APP was not affected by co-transfection with either Abl-PP or SrcYF active kinases (Figure 3A), a reduction in the relative amounts of APP_{sec}, normalised to the corresponding holo-APP synthesised during the pulse period was observed in both cases (Figure 3B). This can be interpreted as a decrease of the activity of the α-secretase pathway upon co-transfection of APP with the active kinases.

### Example 3

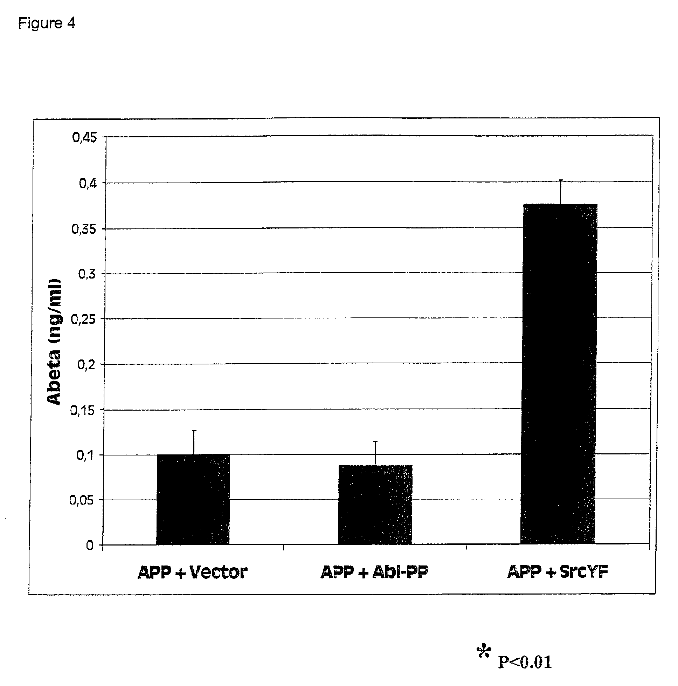

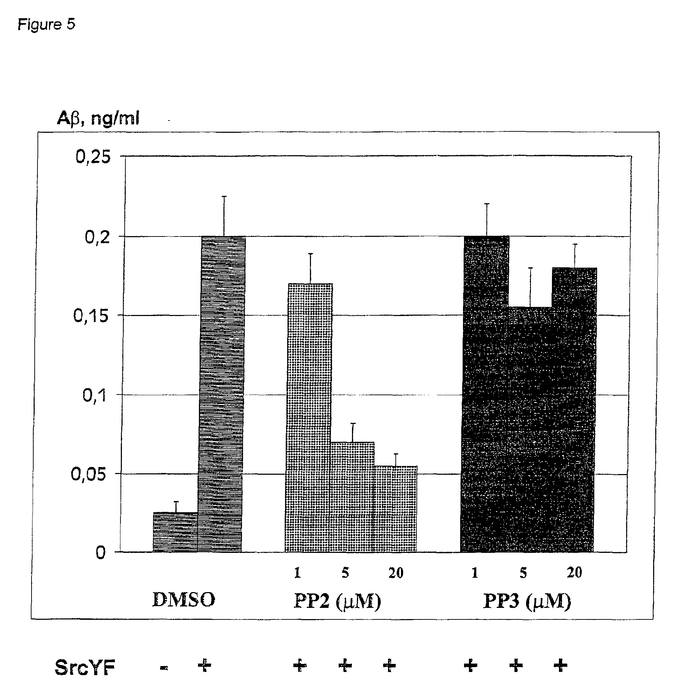

Next, the accumulation of Aβ from cells transfected as above was investigated. The dosage was performed by sandwich ELISA assays on culture medium at 48 hours after transfection (Figure 4). The assay clearly shows that in Src-expressing cells the amount of Aβ secreted is more elevated than in the presence of the control vector, or Abl-PP.
The mechanism by which active Src elicits such a dramatic increase in Aβ secretion is not clear. However, it might depend on phosphorylation of some other proteins, different from APP, since tyrosine phosphorylation of APP by Abl-PP does not result in increased Aβ levels. Accordingly, a similar behaviour is obtained with various APP mutants bearing tyrosine substitutions to either glycine or phenylalanine (data not shown).
Anyway, the rise in Aβ levels does depend on the tyrosine kinase activity of Src, since the accumulation of Aβ is sensitive to treatment by the Src-family specific inhibitor PP2. In fact, as shown in Figure 5, the exposure of transfected cells for 48 hours to increasing concentrations of PP2 results in dose-dependent decrease of secreted Aβ. Either PP3, an inactive analog of PP2, or vehicle alone, do not affect Aβ rise by SrcYF transfection.

Taken together, these observations suggest that exogenous Src activity is responsible for activation of a so far unidentified pathway resulting in increased Aβ secretion.

### REFERENCES

1) T. Russo et al. (1998) FEBS Lett. 434, 1-7.
2) N. Zambrano et al. (1997) J. Biol. Chem. 272, 6399-6405.
3) Sabo S. et al. (1999) J. Biol. Chem. 274, 7952-7957.
4) Borg J.P. et al. (1998) J. Biol. Chem. 273, 14761-14766.
5) Iijima, K. et al. (2000) J. Neurochem. 75, 1085-1091.
6) Suzuki, T. et al. (1994) EMBO J. 13, 1114-1122.
7) Oishi, M. et al. (1997) Mol. Med. 3, 111-123.
8) Aplin, E. E. et al. (1996) J. Neurochem. 67, 699-707.
9) Standen, C. L. et al. (2001) J. Neurochem. 76, 316-320.
10) Ando, K. et al. (1999) J. Neurosci. 19, 4421-4427.
11) Ando K. et al. (2001) J Biol Chem.;276, 40353-40361.
12) Zambrano N. et al. (2001) J Biol Chem 276, 19787-19792.
13) Hanke, J.H. et al. (1996) J. Biol. Chem. 271, 695-701.

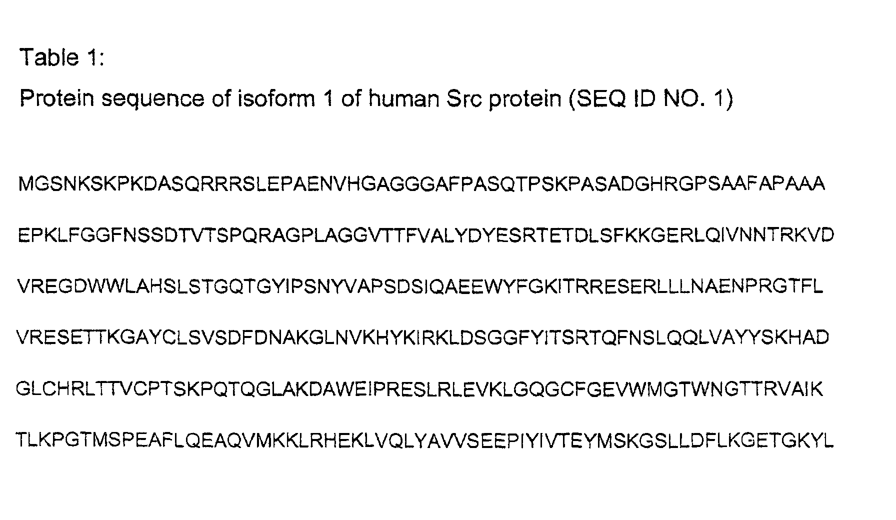

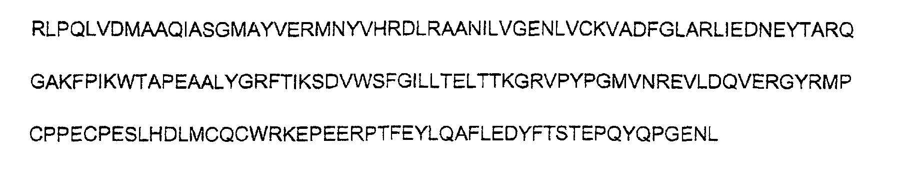

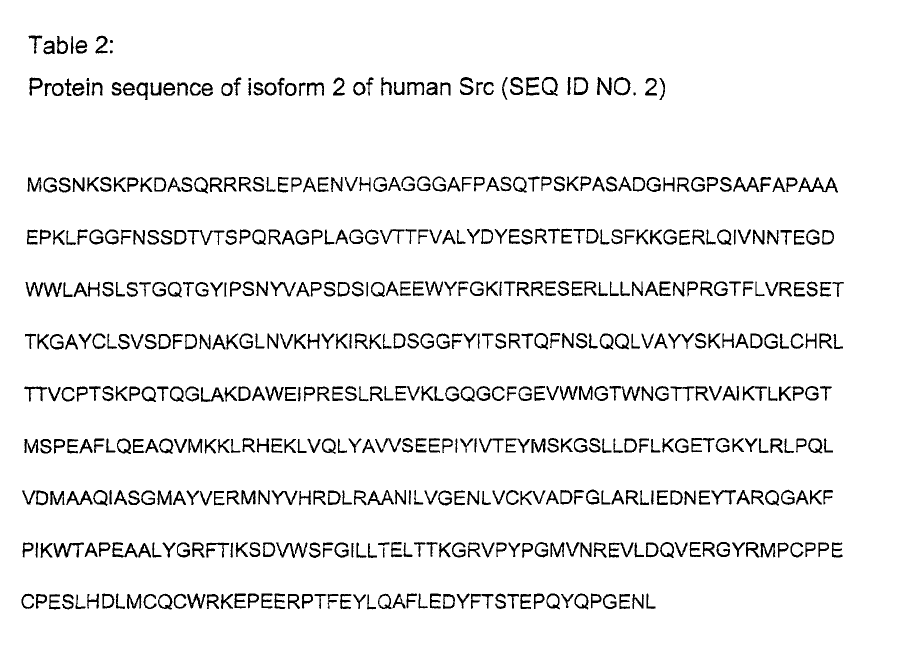

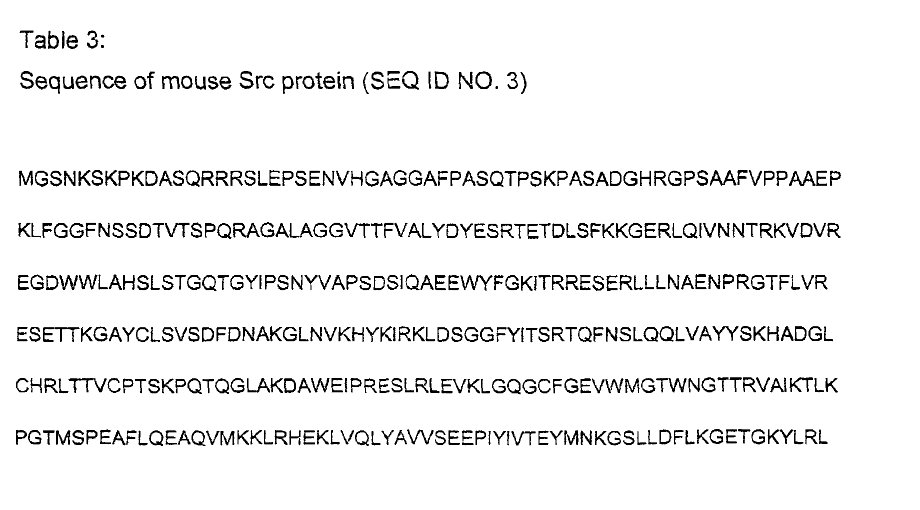

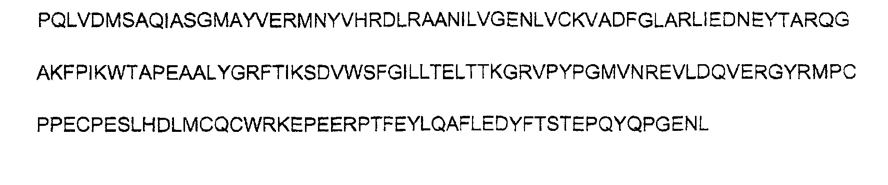

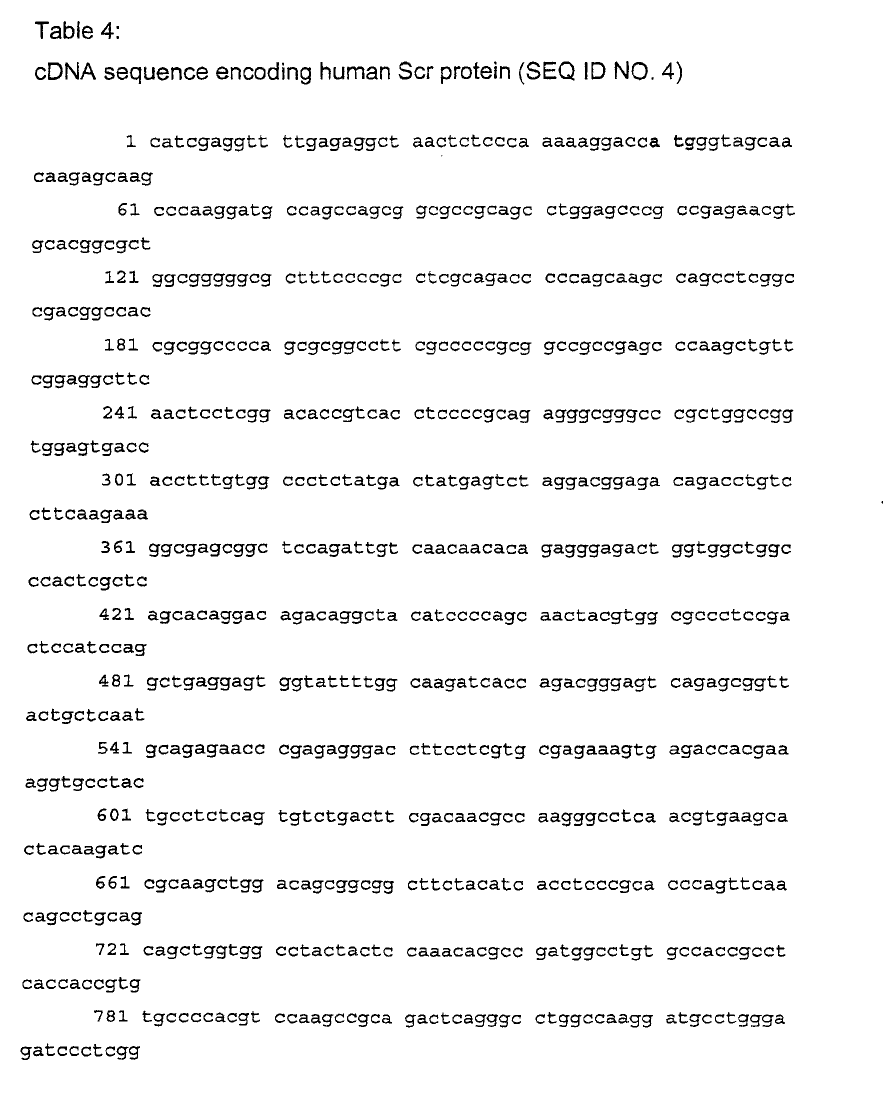

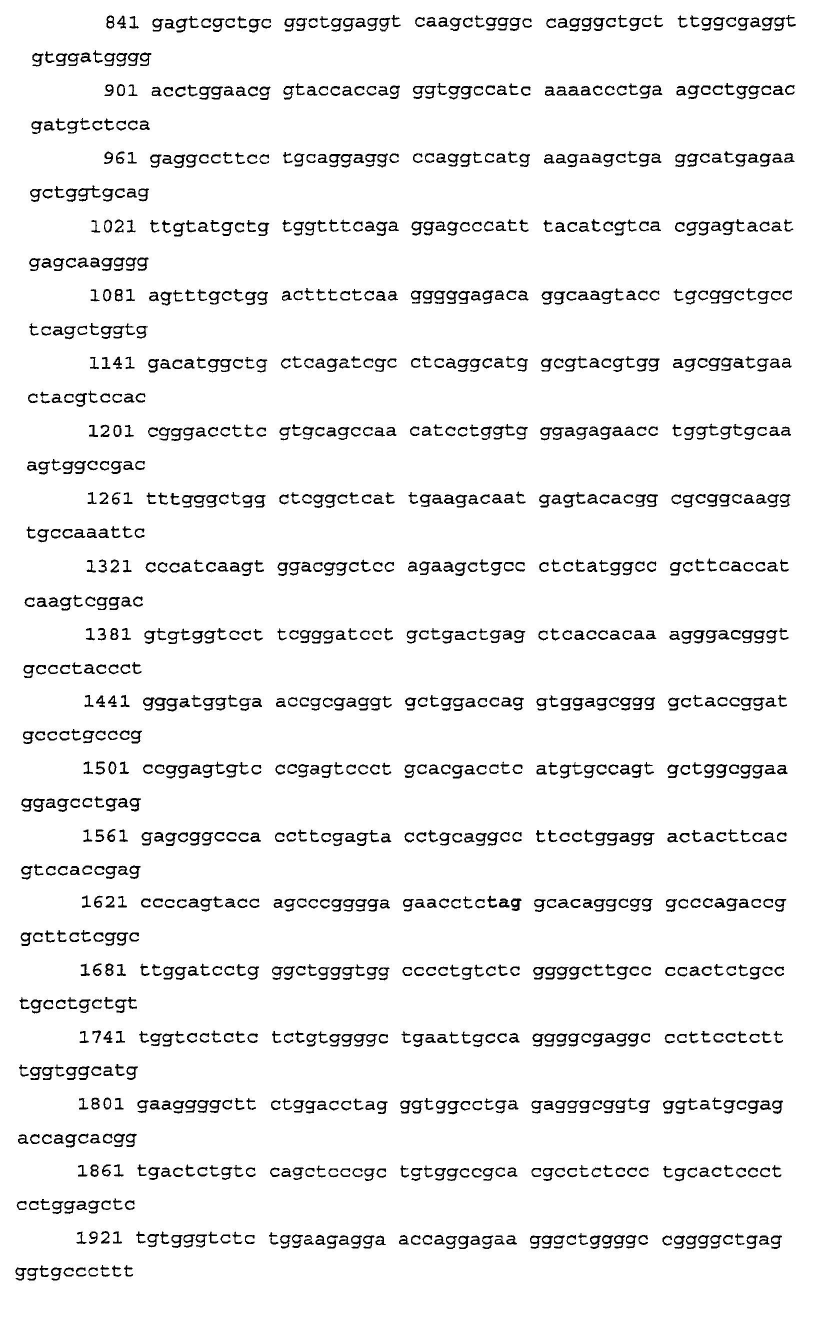

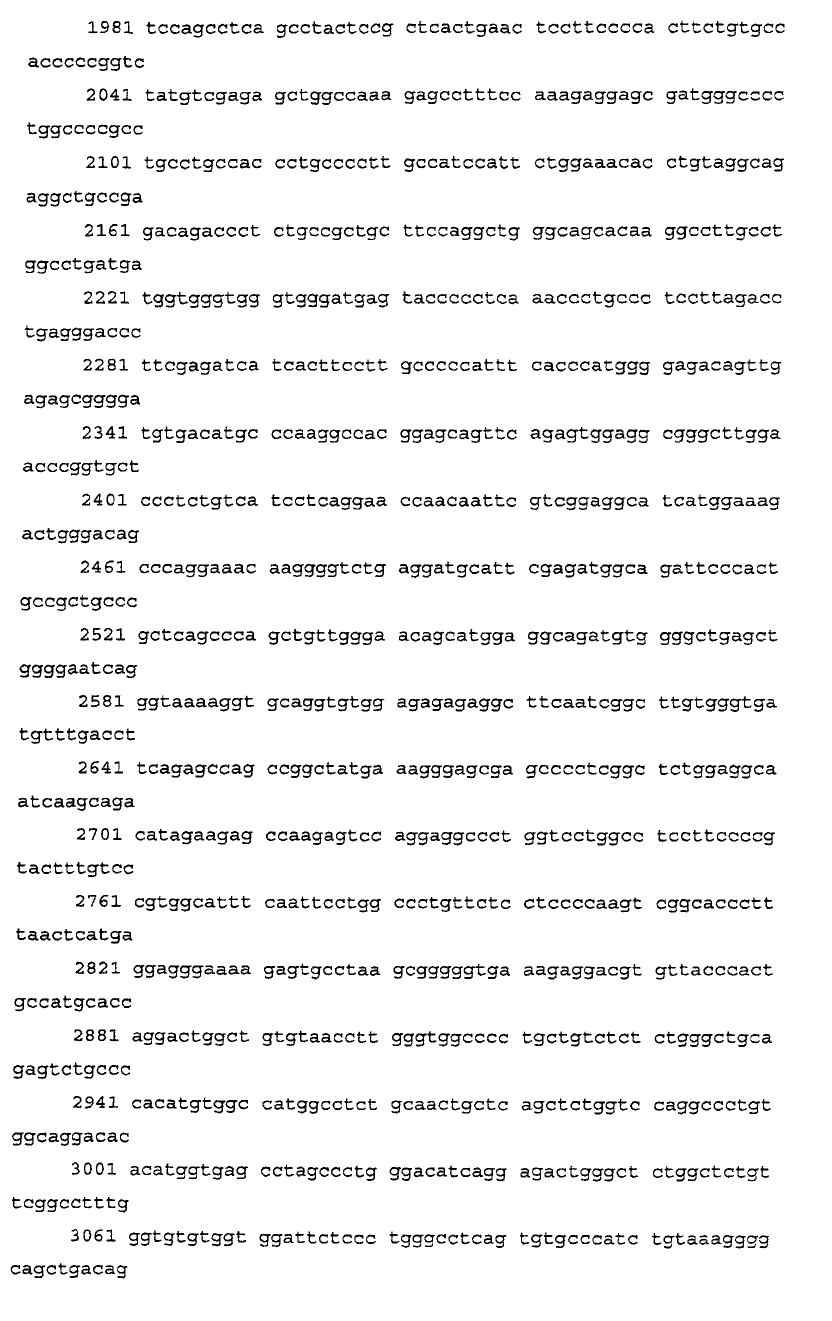

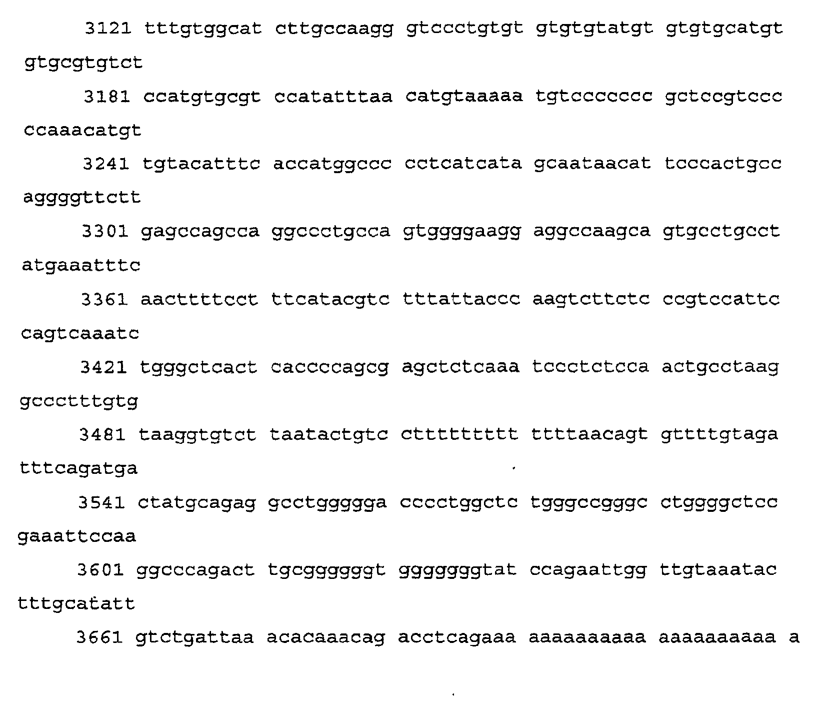

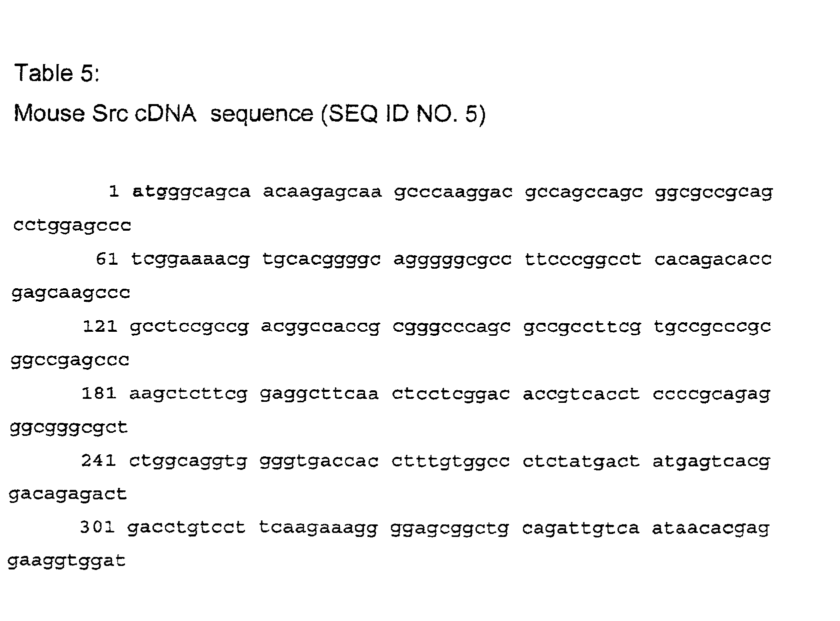

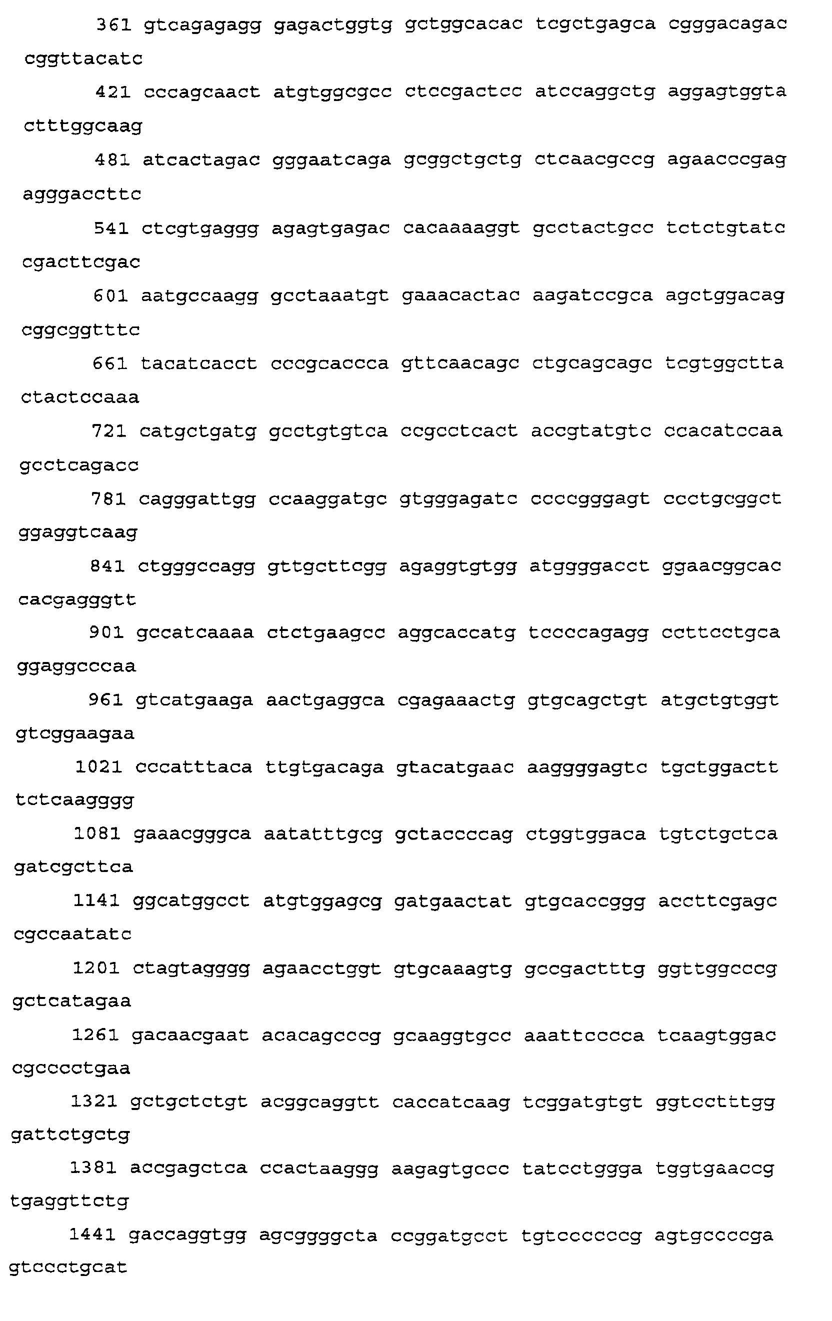

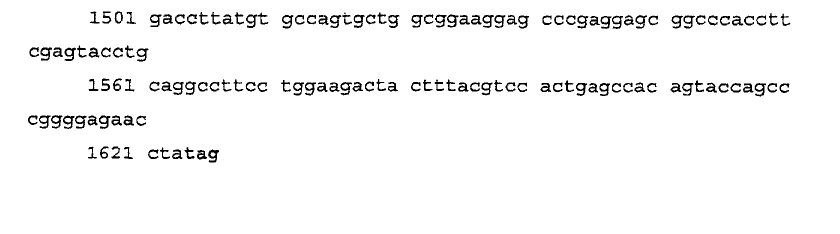

Organization Applicant
---------------------
Street : 20, Avenue Raymond Aron
City : Antony
State : 92160
Country :
PostalCode :
PhoneNumber : 0033 1 55 71 4457
FaxNumber : 0033 1 5571 6172
EmailAddress : Claudia.Meinken@Aventis.com
<110> OrganizationName : Aventis Pharma S. A.

Application Project
-------------------
<120> Title : Inhibitors of Src kinase for use in Alzheimer's disease
<130> AppFileReference : FRAV2002/0030
<140> CurrentAppNumber :
<141> CurrentFilingDate : ____-__-__

Sequence
--------
<213> OrganismName : Homo sapiens
<400> PreSequenceString :
MGSNKSKPKD ASQRRRSLEP AENVHGAGGG AFPASQTPSK PASADGHRGP SAAFAPAAAE 60

PKLFGGFNSS DTVTSPQRAG PLAGGVTTFV ALYDYESRTE TDLSFKKGER LQIVNNTRKV 120

DVREGDWWLA HSLSTGQTGY IPSNYVAPSD SIQAEEWYFG KITRRESERL LLNAENPRGT 180

FLVRESETTK GAYCLSVSDF DNAKGLNVKH YKIRKLDSGG FYITSRTQFN SLQQLVAYYS 240

KHADGLCHRL TTVCPTSKPQ TQGLAKDAWE IPRESLRLEV KLGQGCFGEV WMGTWNGTTR 300

VAIKTLKPGT MSPEAFLQEA QVMKKLRHEK LVQLYAVVSE EPIYIVTEYM SKGSLLDFLK 360

GETGKYLRLP QLVDMAAQIA SGMAYVERMN YVHRDLRAAN ILVGENLVCK VADFGLARLI 420

EDNEYTARQG AKFPIKWTAP EAALYGRFTI KSDVWSFGIL LTELTTKGRV PYPGMVNREV 480

LDQVERGYRM PCPPECPESL HDLMCQCWRK EPEERPTFEY LQAFLEDYFT STEPQYQPGE 540

NL 542
<212> Type : PRT
<211> Length : 542
SequenceName : human src isoform 1
SequenceDescription :

Feature
-------
Sequence: human src isoform 1:
<221> FeatureKey : PEPTIDE
<222> LocationFrom : 1
<222> LocationTo : 542
Other Information :
CDSJoin : No

Sequence
--------
<213> OrganismName : Homo sapiens
<400> PreSequenceString :
 60

 120

 180

 240

 300

 360

 420

 480

 536

<212> Type : PRT
<211> Length : 536
SequenceName : human src isoform 2
SequenceDescription :

Feature
-------
Sequence: human src isoform 2:
<221> FeatureKey : PEPTIDE
<222> LocationFrom : 1
<222> LocationTo : 536
Other Information :
CDSJoin : No

Sequence
--------
<213> OrganismName : Murinae gen. sp.
<400> PreSequenceString :
MGSNKSKPKD ASQRRRSLEP SENVHGAGGA FPASQTPSKP ASADGHRGPS AAFVPPAAEP 60

KLFGGFNSSD TVTSPQRAGA LAGGVTTFVA LYDYESRTET DLSFKKGERL QIVNNTRKVD 120

VREGDWWLAH SLSTGQTGYI PSNYVAPSDS IQAEEWYFGK ITRRESERLL LNAENPRGTF 180

LVRESETTKG AYCLSVSDFD NAKGLNVKHY KIRKLDSGGF YITSRTQFNS LQQLVAYYSK 240

HADGLCHRLT TVCPTSKPQT QGLAKDAWEI PRESLRLEVK LGQGCFGEVW MGTWNGTTRV 300

AIKTLKPGTM SPEAFLQEAQ VMKKLRHEKL VQLYAVVSEE PIYIVTEYMN KGSLLDFLKG 360

ETGKYLRLPQ LVDMSAQIAS GMAYVERMNY VHRDLRAANI LVGENLVCKV ADFGLARLIE 420

DNEYTARQGA KFPIKWTAPE AALYGRFTIK SDVWSFGILL TELTTKGRVP YPGMVNREVL 480

DQVERGYRMP CPPECPESLH DLMCQCWRKE PEERPTFEYL QAFLEDYFTS TEPQYQPGEN 540

L 541
<212> Type : PRT
<211> Length : 541
SequenceName : mouse scr
SequenceDescription :

Feature
-------
Sequence: mouse scr:
<221> FeatureKey : PEPTIDE
<222> LocationFrom : 1
<222> LocationTo : 541
Other Information :
CDSJoin : No

Sequence
--------
<213> OrganismName : Homo sapiens
<400> PreSequenceString :
catcgaggtt ttgagaggct aactctccca aaaaggacca tgggtagcaa caagagcaag 60

cccaaggatg ccagccagcg gcgccgcagc ctggagcccg ccgagaacgt gcacggcgct 120
ggcgggggcg ctttccccgc ctcgcagacc cccagcaagc cagcctcggc cgacggccac 180
cgcggcccca gcgcggcctt cgcccccgcg gccgccgagc ccaagctgtt cggaggcttc 240
aactcctcgg acaccgtcac ctccccgcag agggcgggcc cgctggccgg tggagtgacc 300
acctttgtgg ccctctatga ctatgagtct aggacggaga cagacctgtc cttcaagaaa 360
ggcgagcggc tccagattgt caacaacaca gagggagact ggtggctggc ccactcgctc 420
agcacaggac agacaggcta catccccagc aactacgtgg cgccctccga ctccatccag 480
gctgaggagt ggtattttgg caagatcacc agacgggagt cagagcggtt actgctcaat 540
gcagagaacc cgagagggac cttcctcgtg cgagaaagtg agaccacgaa aggtgcctac 600
tgcctctcag tgtctgactt cgacaacgcc aagggcctca acgtgaagca ctacaagatc 660
cgcaagctgg acagcggcgg cttctacatc acctcccgca cccagttcaa cagcctgcag 720
cagctggtgg cctactactc caaacacgcc gatggcctgt gccaccgcct caccaccgtg 780
tgccccacgt ccaagccgca gactcagggc ctggccaagg atgcctggga gatccctcgg 840
gagtcgctgc ggctggaggt caagctgggc cagggctgct ttggcgaggt gtggatgggg 900
acctggaacg gtaccaccag ggtggccatc aaaaccctga agcctggcac gatgtctcca 960
gaggccttcc tgcaggaggc ccaggtcatg aagaagctga ggcatgagaa gctggtgcag 1020
ttgtatgctg tggtttcaga ggagcccatt tacatcgtca cggagtacat gagcaagggg 1080
agtttgctgg actttctcaa gggggagaca ggcaagtacc tgcggctgcc tcagctggtg 1140
gacatggctg ctcagatcgc ctcaggcatg gcgtacgtgg agcggatgaa ctacgtccac 1200
cgggaccttc gtgcagccaa catcctggtg ggagagaacc tggtgtgcaa agtggccgac 1260
tttgggctgg ctcggctcat tgaagacaat gagtacacgg cgcggcaagg tgccaaattc 1320
cccatcaagt ggacggctcc agaagctgcc ctctatggcc gcttcaccat caagtcggac 1380
gtgtggtcct tcgggatcct gctgactgag ctcaccacaa agggacgggt gccctaccct 1440
gggatggtga accgcgaggt gctggaccag gtggagcggg gctaccggat gccctgcccg 1500
ccggagtgtc ccgagtccct gcacgacctc atgtgccagt gctggcggaa ggagcctgag 1560
gagcggccca ccttcgagta cctgcaggcc ttcctggagg actacttcac gtccaccgag 1620
ccccagtacc agcccgggga gaacctctag gcacaggcgg gcccagaccg gcttctcggc 1680
ttggatcctg ggctgggtgg cccctgtctc ggggcttgcc ccactctgcc tgcctgctgt 1740
tggtcctctc tctgtggggc tgaattgcca ggggcgaggc ccttcctctt tggtggcatg 1800
gaaggggctt ctggacctag ggtggcctga gagggcggtg ggtatgcgag accagcacgg 1860
tgactctgtc cagctcccgc tgtggccgca cgcctctccc tgcactccct cctggagctc 1920
tgtgggtctc tggaagagga accaggagaa gggctggggc cggggctgag ggtgcccttt 1980
tccagcctca gcctactccg ctcactgaac tccttcccca cttctgtgcc acccccggtc 2040
tatgtcgaga gctggccaaa gagcctttcc aaagaggagc gatgggcccc tggccccgcc 2100

tgcctgccac cctgcccctt gccatccatt ctggaaacac ctgtaggcag aggctgccga 2160
gacagaccct ctgccgctgc ttccaggctg ggcagcacaa ggccttgcct ggcctgatga 2220
tggtgggtgg gtgggatgag taccccctca aaccctgccc tccttagacc tgagggaccc 2280
ttcgagatca tcacttcctt gcccccattt cacccatggg gagacagttg agagcgggga 2340
tgtgacatgc ccaaggccac ggagcagttc agagtggagg cgggcttgga acccggtgct 2400
ccctctgtca tcctcaggaa ccaacaattc gtcggaggca tcatggaaag actgggacag 2460
cccaggaaac aaggggtctg aggatgcatt cgagatggca gattcccact gccgctgccc 2520
gctcagccca gctgttggga acagcatgga ggcagatgtg gggctgagct ggggaatcag 2580
ggtaaaaggt gcaggtgtgg agagagaggc ttcaatcggc ttgtgggtga tgtttgacct 2640
tcagagccag ccggctatga aagggagcga gcccctcggc tctggaggca atcaagcaga 2700
catagaagag ccaagagtcc aggaggccct ggtcctggcc tccttccccg tactttgtcc 2760
cgtggcattt caattcctgg ccctgttctc ctccccaagt cggcaccctt taactcatga 2820
ggagggaaaa gagtgcctaa gcgggggtga aagaggacgt gttacccact gccatgcacc 2880
aggactggct gtgtaacctt gggtggcccc tgctgtctct ctgggctgca gagtctgccc 2940
cacatgtggc catggcctct gcaactgctc agctctggtc caggccctgt ggcaggacac 3000
acatggtgag cctagccctg ggacatcagg agactgggct ctggctctgt tcggcctttg 3060
ggtgtgtggt ggattctccc tgggcctcag tgtgcccatc tgtaaagggg cagctgacag 3120
tttgtggcat cttgccaagg gtccctgtgt gtgtgtatgt gtgtgcatgt gtgcgtgtct 3180
ccatgtgcgt ccatatttaa catgtaaaaa tgtccccccc gctccgtccc ccaaacatgt 3240
tgtacatttc accatggccc cctcatcata gcaataacat tcccactgcc aggggttctt 3300
gagccagcca ggccctgcca gtggggaagg aggccaagca gtgcctgcct atgaaatttc 3360
aacttttcct ttcatacgtc tttattaccc aagtcttctc ccgtccattc cagtcaaatc 3420
tgggctcact caccccagcg agctctcaaa tccctctcca actgcctaag gccctttgtg 3480
taaggtgtct taatactgtc cttttttttt ttttaacagt gttttgtaga tttcagatga 3540
ctatgcagag gcctggggga cccctggctc tgggccgggc ctggggctcc gaaattccaa 3600
ggcccagact tgcggggggt gggggggtat ccagaattgg ttgtaaatac tttgcatatt 3660
gtctgattaa acacaaacag acctcagaaa aaaaaaaaaa aaaaaaaaaa a 3711

<212> Type : DNA
<211> Length : 3711
      SequenceName : human src
      SequenceDescription :

Feature
-------
Sequence: human src:
<221> FeatureKey : CDS
<222> LocationFrom : 1
<222> LocationTo : 3711
      Other Information :
      CDSJoin : No

Custom Codon
------------
Sequence Name : human src

Sequence
--------
<213> OrganismName : Murinae gen. sp.
<400> PreSequenceString :
atgggcagca acaagagcaa gcccaaggac gccagccagc ggcgccgcag cctggagccc 60

tcggaaaacg tgcacggggc agggggcgcc ttcccggcct cacagacacc gagcaagccc 120

gcctccgccg acggccaccg cgggcccagc gccgccttcg tgccgcccgc ggccgagccc 180

aagctcttcg gaggcttcaa ctcctcggac accgtcacct ccccgcagag ggcgggcgct 240

ctggcaggtg gggtgaccac ctttgtggcc ctctatgact atgagtcacg gacagagact 300

gacctgtcct tcaagaaagg ggagcggctg cagattgtca ataacacgag gaaggtggat 360

gtcagagagg gagactggtg gctggcacac tcgctgagca cgggacagac cggttacatc 420

cccagcaact atgtggcgcc ctccgactcc atccaggctg aggagtggta ctttggcaag 480

atcactagac gggaatcaga gcggctgctg ctcaacgccg agaacccgag agggaccttc 540

ctcgtgaggg agagtgagac cacaaaaggt gcctactgcc tctctgtatc cgacttcgac 600

aatgccaagg gcctaaatgt gaaacactac aagatccgca agctggacag cggcggtttc 660

tacatcacct cccgcaccca gttcaacagc ctgcagcagc tcgtggctta ctactccaaa 720

catgctgatg gcctgtgtca ccgcctcact accgtatgtc ccacatccaa gcctcagacc 780

cagggattgg ccaaggatgc gtgggagatc ccccgggagt ccctgcggct ggaggtcaag 840

ctgggccagg gttgcttcgg agaggtgtgg atggggacct ggaacggcac cacgagggtt 900

gccatcaaaa ctctgaagcc aggcaccatg tccccagagg ccttcctgca ggaggcccaa 960

gtcatgaaga aactgaggca cgagaaactg gtgcagctgt atgctgtggt gtcggaagaa 1020

cccatttaca ttgtgacaga gtacatgaac aaggggagtc tgctggactt tctcaagggg 1080

gaaacgggca aatatttgcg gctaccccag ctggtggaca tgtctgctca gatcgcttca 1140

ggcatggcct atgtggagcg gatgaactat gtgcaccggg accttcgagc cgccaatatc 1200

ctagtagggg agaacctggt gtgcaaagtg gccgactttg ggttggcccg gctcatagaa 1260

gacaacgaat acacagcccg gcaaggtgcc aaattcccca tcaagtggac cgcccctgaa 1320

gctgctctgt acggcaggtt caccatcaag tcggatgtgt ggtcctttgg gattctgctg 1380

accgagctca ccactaaggg aagagtgccc tatcctggga tggtgaaccg tgaggttctg 1440

gaccaggtgg agcggggcta ccggatgcct tgtccccccg agtgccccga gtccctgcat 1500

gaccttatgt gccagtgctg gcggaaggag cccgaggagc ggcccacctt cgagtacctg 1560

caggccttcc tggaagacta ctttacgtcc actgagccac agtaccagcc cggggagaac 1620

ctatag 1626
<212> Type : DNA
<211> Length : 1626
SequenceName : Mouse src
SequenceDescription :

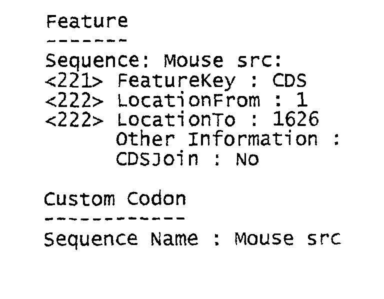

SEQUENCE LISTING

<110> Aventis S.A.

<120> Inhibitors of Src kinase for use in Alzheimer's disease

<130> FRAV2002/0030

<140> EP 02292608.3
<141> 2002-10-31

<160> 5

<170> PatentIn version 3.1

<210> 1
<211> 542
<212> PRT
<213> Homo sapiens

<220>
<221> PEPTIDE
<222> (1)..(542)
<223>

<400> 1

Met Gly Ser Asn Lys Ser Lys Pro Lys Asp Ala Ser Gln Arg Arg Arg
1               5                   10                  15

Ser Leu Glu Pro Ala Glu Asn Val His Gly Ala Gly Gly Gly Ala Phe
            20                  25                  30

Pro Ala Ser Gln Thr Pro Ser Lys Pro Ala Ser Ala Asp Gly His Arg
        35                  40                  45

Gly Pro Ser Ala Ala Phe Ala Pro Ala Ala Ala Glu Pro Lys Leu Phe
    50                  55                  60

Gly Gly Phe Asn Ser Ser Asp Thr Val Thr Ser Pro Gln Arg Ala Gly
65                  70                  75                  80

Pro Leu Ala Gly Gly Val Thr Thr Phe Val Ala Leu Tyr Asp Tyr Glu
                85                  90                  95

Ser Arg Thr Glu Thr Asp Leu Ser Phe Lys Lys Gly Glu Arg Leu Gln
            100                 105                 110

Ile Val Asn Asn Thr Arg Lys Val Asp Val Arg Glu Gly Asp Trp Trp
        115                 120                 125

Leu Ala His Ser Leu Ser Thr Gly Gln Thr Gly Tyr Ile Pro Ser Asn
    130                 135                 140

Tyr Val Ala Pro Ser Asp Ser Ile Gln Ala Glu Glu Trp Tyr Phe Gly
145                 150                 155                 160

Lys Ile Thr Arg Arg Glu Ser Glu Arg Leu Leu Leu Asn Ala Glu Asn
                165                 170                 175

Pro Arg Gly Thr Phe Leu Val Arg Glu Ser Glu Thr Thr Lys Gly Ala
            180                 185                 190

Tyr Cys Leu Ser Val Ser Asp Phe Asp Asn Ala Lys Gly Leu Asn Val
        195                 200                 205

Lys His Tyr Lys Ile Arg Lys Leu Asp Ser Gly Gly Phe Tyr Ile Thr
    210                 215                 220

Ser Arg Thr Gln Phe Asn Ser Leu Gln Gln Leu Val Ala Tyr Tyr Ser
225                 230                 235                 240

Lys His Ala Asp Gly Leu Cys His Arg Leu Thr Thr Val Cys Pro Thr
                245                 250                 255

Ser Lys Pro Gln Thr Gln Gly Leu Ala Lys Asp Ala Trp Glu Ile Pro
            260                 265                 270

Arg Glu Ser Leu Arg Leu Glu Val Lys Leu Gly Gln Gly Cys Phe Gly
        275                 280                 285

Glu Val Trp Met Gly Thr Trp Asn Gly Thr Thr Arg Val Ala Ile Lys
    290                 295                 300

Thr Leu Lys Pro Gly Thr Met Ser Pro Glu Ala Phe Leu Gln Glu Ala
305                 310                 315                 320

Gln Val Met Lys Lys Leu Arg His Glu Lys Leu Val Gln Leu Tyr Ala
                325                 330                 335

Val Val Ser Glu Glu Pro Ile Tyr Ile Val Thr Glu Tyr Met Ser Lys
            340                 345                 350

Gly Ser Leu Leu Asp Phe Leu Lys Gly Glu Thr Gly Lys Tyr Leu Arg
        355                 360                 365

Leu Pro Gln Leu Val Asp Met Ala Ala Gln Ile Ala Ser Gly Met Ala
    370                 375                 380

Tyr Val Glu Arg Met Asn Tyr Val His Arg Asp Leu Arg Ala Ala Asn
385                 390                 395                 400

Ile Leu Val Gly Glu Asn Leu Val Cys Lys Val Ala Asp Phe Gly Leu
                405                 410                 415

Ala Arg Leu Ile Glu Asp Asn Glu Tyr Thr Ala Arg Gln Gly Ala Lys
            420                 425                 430

Phe Pro Ile Lys Trp Thr Ala Pro Glu Ala Ala Leu Tyr Gly Arg Phe
        435                 440                 445

Thr Ile Lys Ser Asp Val Trp Ser Phe Gly Ile Leu Leu Thr Glu Leu
    450                 455                 460

Thr Thr Lys Gly Arg Val Pro Tyr Pro Gly Met Val Asn Arg Glu Val
465                 470                 475                 480

Leu Asp Gln Val Glu Arg Gly Tyr Arg Met Pro Cys Pro Pro Glu Cys
                485                 490                 495

Pro Glu Ser Leu His Asp Leu Met Cys Gln Cys Trp Arg Lys Glu Pro
            500                 505                 510

Glu Glu Arg Pro Thr Phe Glu Tyr Leu Gln Ala Phe Leu Glu Asp Tyr
        515                 520                 525

Phe Thr Ser Thr Glu Pro Gln Tyr Gln Pro Gly Glu Asn Leu
    530                 535                 540

<210>  2

<211>  536

<212>  PRT

<213>  Homo sapiens


<220>

<221>  PEPTIDE

<222>  (1)..(536)

<223>

<400> 2

<210>  3

<211>  541

<212>  PRT

<213>  Murinae gen. sp.


<220>

<221>  PEPTIDE

<222>  (1)..(541)

<223>


<400>  3

Met Gly Ser Asn Lys Ser Lys Pro Lys Asp Ala Ser Gln Arg Arg Arg
1               5                   10                  15

Ser Leu Glu Pro Ser Glu Asn Val His Gly Ala Gly Gly Ala Phe Pro
            20                  25                  30

Ala Ser Gln Thr Pro Ser Lys Pro Ala Ser Ala Asp Gly His Arg Gly
        35                  40                  45

Pro Ser Ala Ala Phe Val Pro Pro Ala Ala Glu Pro Lys Leu Phe Gly
    50                  55                  60

Gly Phe Asn Ser Ser Asp Thr Val Thr Ser Pro Gln Arg Ala Gly Ala
65                  70                  75                  80

Leu Ala Gly Gly Val Thr Thr Phe Val Ala Leu Tyr Asp Tyr Glu Ser
                85                  90                  95

Arg Thr Glu Thr Asp Leu Ser Phe Lys Lys Gly Glu Arg Leu Gln Ile
            100                 105                 110

Val Asn Asn Thr Arg Lys Val Asp Val Arg Glu Gly Asp Trp Trp Leu
        115                 120                 125

Ala His Ser Leu Ser Thr Gly Gln Thr Gly Tyr Ile Pro Ser Asn Tyr
    130                 135                 140

Val Ala Pro Ser Asp Ser Ile Gln Ala Glu Glu Trp Tyr Phe Gly Lys
145                 150                 155                 160

Ile Thr Arg Arg Glu Ser Glu Arg Leu Leu Leu Asn Ala Glu Asn Pro
                165                 170                 175

Arg Gly Thr Phe Leu Val Arg Glu Ser Glu Thr Thr Lys Gly Ala Tyr
            180                 185                 190

Cys Leu Ser Val Ser Asp Phe Asp Asn Ala Lys Gly Leu Asn Val Lys
        195                 200                 205

His Tyr Lys Ile Arg Lys Leu Asp Ser Gly Gly Phe Tyr Ile Thr Ser
    210                 215                 220

Arg Thr Gln Phe Asn Ser Leu Gln Gln Leu Val Ala Tyr Tyr Ser Lys
225                 230                 235                 240

His Ala Asp Gly Leu Cys His Arg Leu Thr Thr Val Cys Pro Thr Ser
                245                 250                 255

Lys Pro Gln Thr Gln Gly Leu Ala Lys Asp Ala Trp Glu Ile Pro Arg
            260                 265                 270

Glu Ser Leu Arg Leu Glu Val Lys Leu Gly Gln Gly Cys Phe Gly Glu
        275                 280                 285

Val Trp Met Gly Thr Trp Asn Gly Thr Thr Arg Val Ala Ile Lys Thr
    290                 295                 300

Leu Lys Pro Gly Thr Met Ser Pro Glu Ala Phe Leu Gln Glu Ala Gln
305                 310                 315                 320

Val Met Lys Lys Leu Arg His Glu Lys Leu Val Gln Leu Tyr Ala Val
                325                 330                 335

Val Ser Glu Glu Pro Ile Tyr Ile Val Thr Glu Tyr Met Asn Lys Gly
            340                 345                 350

Ser Leu Leu Asp Phe Leu Lys Gly Glu Thr Gly Lys Tyr Leu Arg Leu
        355                 360                 365

Pro Gln Leu Val Asp Met Ser Ala Gln Ile Ala Ser Gly Met Ala Tyr
    370                 375                 380

Val Glu Arg Met Asn Tyr Val His Arg Asp Leu Arg Ala Ala Asn Ile
385                 390                 395                 400

Leu Val Gly Glu Asn Leu Val Cys Lys Val Ala Asp Phe Gly Leu Ala
                405                 410                 415

Arg Leu Ile Glu Asp Asn Glu Tyr Thr Ala Arg Gln Gly Ala Lys Phe
            420                 425                 430

Pro Ile Lys Trp Thr Ala Pro Glu Ala Ala Leu Tyr Gly Arg Phe Thr
        435                 440                 445

Ile Lys Ser Asp Val Trp Ser Phe Gly Ile Leu Leu Thr Glu Leu Thr
    450                 455                 460

Thr Lys Gly Arg Val Pro Tyr Pro Gly Met Val Asn Arg Glu Val Leu
465                 470                 475                 480

Asp Gln Val Glu Arg Gly Tyr Arg Met Pro Cys Pro Pro Glu Cys Pro
                485                 490                 495

Glu Ser Leu His Asp Leu Met Cys Gln Cys Trp Arg Lys Glu Pro Glu
            500                 505                 510

Glu Arg Pro Thr Phe Glu Tyr Leu Gln Ala Phe Leu Glu Asp Tyr Phe
        515                 520                 525

Thr Ser Thr Glu Pro Gln Tyr Gln Pro Gly Glu Asn Leu
    530                 535                 540

<210>  4

<211>  3711

<212>  DNA

<213>  Homo sapiens


<220>

<221>  exon

<222>  (1)..(3711)

<223>


<400>  4
cat cga ggt ttt gag agg cta act ctc cca aaa agg acc atg ggt agc        48
His Arg Gly Phe Glu Arg Leu Thr Leu Pro Lys Arg Thr Met Gly Ser
1               5                   10                  15

aac aag agc aag ccc aag gat gcc agc cag cgg cgc cgc agc ctg gag        96
Asn Lys Ser Lys Pro Lys Asp Ala Ser Gln Arg Arg Arg Ser Leu Glu
            20                  25                  30

ccc gcc gag aac gtg cac ggc gct ggc ggg ggc gct ttc ccc gcc tcg       144
Pro Ala Glu Asn Val His Gly Ala Gly Gly Gly Ala Phe Pro Ala Ser
        35                  40                  45

cag acc ccc agc aag cca gcc tcg gcc gac ggc cac cgc ggc ccc agc       192
Gln Thr Pro Ser Lys Pro Ala Ser Ala Asp Gly His Arg Gly Pro Ser
    50                  55                  60

gcg gcc ttc gcc ccc gcg gcc gcc gag ccc aag ctg ttc gga ggc ttc       240
Ala Ala Phe Ala Pro Ala Ala Ala Glu Pro Lys Leu Phe Gly Gly Phe
65                  70                  75                  80

aac tcc tcg gac acc gtc acc tcc ccg cag agg gcg ggc ccg ctg gcc 288
Asn Ser Ser Asp Thr Val Thr Ser Pro Gln Arg Ala Gly Pro Leu Ala
                85                  90                  95

ggt gga gtg acc acc ttt gtg gcc ctc tat gac tat gag tct agg acg 336
Gly Gly Val Thr Thr Phe Val Ala Leu Tyr Asp Tyr Glu Ser Arg Thr
            100                 105                 110

gag aca gac ctg tcc ttc aag aaa ggc gag cgg ctc cag att gtc aac 384
Glu Thr Asp Leu Ser Phe Lys Lys Gly Glu Arg Leu Gln Ile Val Asn
        115                 120                 125

aac aca gag gga gac tgg tgg ctg gcc cac tcg ctc agc aca gga cag 432
Asn Thr Glu Gly Asp Trp Trp Leu Ala His Ser Leu Ser Thr Gly Gln
    130                 135                 140

aca ggc tac atc ccc agc aac tac gtg gcg ccc tcc gac tcc atc cag 480
Thr Gly Tyr Ile Pro Ser Asn Tyr Val Ala Pro Ser Asp Ser Ile Gln
145                 150                 155                 160

gct gag gag tgg tat ttt ggc aag atc acc aga cgg gag tca gag cgg 528
Ala Glu Glu Trp Tyr Phe Gly Lys Ile Thr Arg Arg Glu Ser Glu Arg
                165                 170                 175

tta ctg ctc aat gca gag aac ccg aga ggg acc ttc ctc gtg cga gaa 576
Leu Leu Leu Asn Ala Glu Asn Pro Arg Gly Thr Phe Leu Val Arg Glu
            180                 185                 190

agt gag acc acg aaa ggt gcc tac tgc ctc tca gtg tct gac ttc gac 624
Ser Glu Thr Thr Lys Gly Ala Tyr Cys Leu Ser Val Ser Asp Phe Asp
        195                 200                 205

aac gcc aag ggc ctc aac gtg aag cac tac aag atc cgc aag ctg gac 672
Asn Ala Lys Gly Leu Asn Val Lys His Tyr Lys Ile Arg Lys Leu Asp
    210                 215                 220

agc ggc ggc ttc tac atc acc tcc cgc acc cag ttc aac agc ctg cag 720
Ser Gly Gly Phe Tyr Ile Thr Ser Arg Thr Gln Phe Asn Ser Leu Gln
225                 230                 235                 240

cag ctg gtg gcc tac tac tcc aaa cac gcc gat ggc ctg tgc cac cgc 768
Gln Leu Val Ala Tyr Tyr Ser Lys His Ala Asp Gly Leu Cys His Arg
                245                 250                 255

ctc acc acc gtg tgc ccc acg tcc aag ccg cag act cag ggc ctg gcc 816
Leu Thr Thr Val Cys Pro Thr Ser Lys Pro Gln Thr Gln Gly Leu Ala
            260                 265                 270

aag gat gcc tgg gag atc cct cgg gag tcg ctg cgg ctg gag gtc aag 864
Lys Asp Ala Trp Glu Ile Pro Arg Glu Ser Leu Arg Leu Glu Val Lys
        275                 280                 285

ctg ggc cag ggc tgc ttt ggc gag gtg tgg atg ggg acc tgg aac ggt 912
Leu Gly Gln Gly Cys Phe Gly Glu Val Trp Met Gly Thr Trp Asn Gly
    290                 295                 300

acc acc agg gtg gcc atc aaa acc ctg aag cct ggc acg atg tct cca 960
Thr Thr Arg Val Ala Ile Lys Thr Leu Lys Pro Gly Thr Met Ser Pro
305                 310                 315                 320

gag gcc ttc ctg cag gag gcc cag gtc atg aag aag ctg agg cat gag 1008
Glu Ala Phe Leu Gln Glu Ala Gln Val Met Lys Lys Leu Arg His Glu
                325                 330                 335

aag ctg gtg cag ttg tat gct gtg gtt tca gag gag ccc att tac atc 1056
Lys Leu Val Gln Leu Tyr Ala Val Val Ser Glu Glu Pro Ile Tyr Ile
            340                 345                 350

gtc acg gag tac atg agc aag ggg agt ttg ctg gac ttt ctc aag ggg 1104
Val Thr Glu Tyr Met Ser Lys Gly Ser Leu Leu Asp Phe Leu Lys Gly
        355                 360                 365

gag aca ggc aag tac ctg cgg ctg cct cag ctg gtg gac atg gct gct 1152
Glu Thr Gly Lys Tyr Leu Arg Leu Pro Gln Leu Val Asp Met Ala Ala
    370                 375                 380

cag atc gcc tca ggc atg gcg tac gtg gag cgg atg aac tac gtc cac 1200
Gln Ile Ala Ser Gly Met Ala Tyr Val Glu Arg Met Asn Tyr Val His
385                 390                 395                 400

cgg gac ctt cgt gca gcc aac atc ctg gtg gga gag aac ctg gtg tgc 1248
Arg Asp Leu Arg Ala Ala Asn Ile Leu Val Gly Glu Asn Leu Val Cys
                405                 410                 415

aaa gtg gcc gac ttt ggg ctg gct cgg ctc att gaa gac aat gag tac 1296
Lys Val Ala Asp Phe Gly Leu Ala Arg Leu Ile Glu Asp Asn Glu Tyr
            420                 425                 430

acg gcg cgg caa ggt gcc aaa ttc ccc atc aag tgg acg gct cca gaa 1344
Thr Ala Arg Gln Gly Ala Lys Phe Pro Ile Lys Trp Thr Ala Pro Glu
        435                 440                 445

gct gcc ctc tat ggc cgc ttc acc atc aag tcg gac gtg tgg tcc ttc 1392
Ala Ala Leu Tyr Gly Arg Phe Thr Ile Lys Ser Asp Val Trp Ser Phe
    450                 455                 460

ggg atc ctg ctg act gag ctc acc aca aag gga cgg gtg ccc tac cct 1440
Gly Ile Leu Leu Thr Glu Leu Thr Thr Lys Gly Arg Val Pro Tyr Pro
465                 470                 475                 480

ggg atg gtg aac cgc gag gtg ctg gac cag gtg gag cgg ggc tac cgg 1488
Gly Met Val Asn Arg Glu Val Leu Asp Gln Val Glu Arg Gly Tyr Arg
                485                 490                 495

atg ccc tgc ccg ccg gag tgt ccc gag tcc ctg cac gac ctc atg tgc 1536
Met Pro Cys Pro Pro Glu Cys Pro Glu Ser Leu His Asp Leu Met Cys
            500                 505                 510

cag tgc tgg cgg aag gag cct gag gag cgg ccc acc ttc gag tac ctg 1584
Gln Cys Trp Arg Lys Glu Pro Glu Glu Arg Pro Thr Phe Glu Tyr Leu
        515                 520                 525

cag gcc ttc ctg gag gac tac ttc acg tcc acc gag ccc cag tac cag 1632
Gln Ala Phe Leu Glu Asp Tyr Phe Thr Ser Thr Glu Pro Gln Tyr Gln
    530                 535                 540

ccc ggg gag aac ctc tag gca cag gcg ggc cca gac cgg ctt ctc ggc 1680
Pro Gly Glu Asn Leu Ala Gln Ala Gly Pro Asp Arg Leu Leu Gly
545                 550                 555

ttg gat cct ggg ctg ggt ggc ccc tgt ctc ggg gct tgc ccc act ctg 1728
Leu Asp Pro Gly Leu Gly Gly Pro Cys Leu Gly Ala Cys Pro Thr Leu
560                 565                 570                 575

cct gcc tgc tgt tgg tcc tct ctc tgt ggg gct gaa ttg cca ggg gcg 1776
Pro Ala Cys Cys Trp Ser Ser Leu Cys Gly Ala Glu Leu Pro Gly Ala
                580                 585                 590

agg ccc ttc ctc ttt ggt ggc atg gaa ggg gct tct gga cct agg gtg 1824
Arg Pro Phe Leu Phe Gly Gly Met Glu Gly Ala Ser Gly Pro Arg Val
            595                 600                 605

gcc tga gag ggc ggt ggg tat gcg aga cca gca cgg tga ctc tgt cca 1872
Ala Glu Gly Gly Gly Tyr Ala Arg Pro Ala Arg Leu Cys Pro
        610                 615                 620

gct ccc gct gtg gcc gca cgc ctc tcc ctg cac tcc ctc ctg gag ctc 1920
Ala Pro Ala Val Ala Ala Arg Leu Ser Leu His Ser Leu Leu Glu Leu
            625                 630                 635

tgt ggg tct ctg gaa gag gaa cca gga gaa ggg ctg ggg ccg ggg ctg 1968
Cys Gly Ser Leu Glu Glu Glu Pro Gly Glu Gly Leu Gly Pro Gly Leu
        640                 645                 650

agg gtg ccc ttt tcc agc ctc agc cta ctc cgc tca ctg aac tcc ttc 2016
Arg Val Pro Phe Ser Ser Leu Ser Leu Leu Arg Ser Leu Asn Ser Phe
    655                 660                 665

ccc act tct gtg cca ccc ccg gtc tat gtc gag agc tgg cca aag agc 2064
Pro Thr Ser Val Pro Pro Pro Val Tyr Val Glu Ser Trp Pro Lys Ser
670                 675                 680                 685

ctt tcc aaa gag gag cga tgg gcc cct ggc ccc gcc tgc ctg cca ccc 2112
Leu Ser Lys Glu Glu Arg Trp Ala Pro Gly Pro Ala Cys Leu Pro Pro
                690                 695                 700

tgc ccc ttg cca tcc att ctg gaa aca cct gta ggc aga ggc tgc cga 2160
Cys Pro Leu Pro Ser Ile Leu Glu Thr Pro Val Gly Arg Gly Cys Arg
            705                 710                 715

gac aga ccc tct gcc gct gct tcc agg ctg ggc agc aca agg cct tgc 2208
Asp Arg Pro Ser Ala Ala Ala Ser Arg Leu Gly Ser Thr Arg Pro Cys
        720                 725                 730

ctg gcc tga tga tgg tgg gtg ggt ggg atg agt acc ccc tca aac cct 2256
Leu Ala Trp Trp Val Gly Gly Met Ser Thr Pro Ser Asn Pro
    735                 740                 745

gcc ctc ctt aga cct gag gga ccc ttc gag atc atc act tcc ttg ccc 2304
Ala Leu Leu Arg Pro Glu Gly Pro Phe Glu Ile Ile Thr Ser Leu Pro
        750                 755                 760

cca ttt cac cca tgg gga gac agt tga gag cgg gga tgt gac atg ccc 2352
Pro Phe His Pro Trp Gly Asp Ser Glu Arg Gly Cys Asp Met Pro
    765                 770                 775

aag gcc acg gag cag ttc aga gtg gag gcg ggc ttg gaa ccc ggt gct 2400
Lys Ala Thr Glu Gln Phe Arg Val Glu Ala Gly Leu Glu Pro Gly Ala
    780                 785                 790

ccc tct gtc atc ctc agg aac caa caa ttc gtc gga ggc atc atg gaa 2448
Pro Ser Val Ile Leu Arg Asn Gln Gln Phe Val Gly Gly Ile Met Glu
795                 800                 805                 810

aga ctg gga cag ccc agg aaa caa ggg gtc tga gga tgc att cga gat 2496
Arg Leu Gly Gln Pro Arg Lys Gln Gly Val Gly Cys Ile Arg Asp
                815                 820                 825

ggc aga ttc cca ctg ccg ctg ccc gct cag ccc agc tgt tgg gaa cag 2544
Gly Arg Phe Pro Leu Pro Leu Pro Ala Gln Pro Ser Cys Trp Glu Gln
                830                 835                 840

cat gga ggc aga tgt ggg gct gag ctg ggg aat cag ggt aaa agg tgc 2592
His Gly Gly Arg Cys Gly Ala Glu Leu Gly Asn Gln Gly Lys Arg Cys
            845                 850                 855

agg tgt gga gag aga ggc ttc aat cgg ctt gtg ggt gat gtt tga cct 2640
Arg Cys Gly Glu Arg Gly Phe Asn Arg Leu Val Gly Asp Val Pro
        860                 865                 870

tca gag cca gcc ggc tat gaa agg gag cga gcc cct cgg ctc tgg agg 2688
Ser Glu Pro Ala Gly Tyr Glu Arg Glu Arg Ala Pro Arg Leu Trp Arg
        875                 880                 885

caa tca agc aga cat aga aga gcc aag agt cca gga ggc cct ggt cct 2736
Gln Ser Ser Arg His Arg Arg Ala Lys Ser Pro Gly Gly Pro Gly Pro
    890                 895                 900

ggc ctc ctt ccc cgt act ttg tcc cgt ggc att tca att cct ggc cct 2784
Gly Leu Leu Pro Arg Thr Leu Ser Arg Gly Ile Ser Ile Pro Gly Pro
905                 910                 915                 920

gtt ctc ctc ccc aag tcg gca ccc ttt aac tca tga gga ggg aaa aga 2832
Val Leu Leu Pro Lys Ser Ala Pro Phe Asn Ser Gly Gly Lys Arg
                925                 930                 935

gtg cct aag cgg ggg tga aag agg acg tgt tac cca ctg cca tgc acc 2880
Val Pro Lys Arg Gly Lys Arg Thr Cys Tyr Pro Leu Pro Cys Thr
                940                 945                 950

agg act ggc tgt gta acc ttg ggt ggc ccc tgc tgt ctc tct ggg ctg 2928
Arg Thr Gly Cys Val Thr Leu Gly Gly Pro Cys Cys Leu Ser Gly Leu
                955                 960                 965

cag agt ctg ccc cac atg tgg cca tgg cct ctg caa ctg ctc agc tct 2976
Gln Ser Leu Pro His Met Trp Pro Trp Pro Leu Gln Leu Leu Ser Ser
            970                 975                 980

ggt cca ggc cct gtg gca gga cac aca tgg tga gcc tag ccc tgg gac 3024
Gly Pro Gly Pro Val Ala Gly His Thr Trp Ala Pro Trp Asp
        985                 990                 995

atc agg aga ctg ggc tct ggc tct gtt cgg cct ttg ggt gtg tgg 3069
Ile Arg Arg Leu Gly Ser Gly Ser Val Arg Pro Leu Gly Val Trp
            1000                1005                1010

tgg att ctc cct ggg cct cag tgt gcc cat ctg taa agg ggc agc 3114
Trp Ile Leu Pro Gly Pro Gln Cys Ala His Leu Arg Gly Ser
            1015                1020                1025

tga cag ttt gtg gca tct tgc caa ggg tcc ctg tgt gtg tgt atg 3159
Gln Phe Val Ala Ser Cys Gln Gly Ser Leu Cys Val Cys Met
                1030                1035

tgt gtg cat gtg tgc gtg tct cca tgt gcg tcc ata ttt aac atg 3204
Cys Val His Val Cys Val Ser Pro Cys Ala Ser Ile Phe Asn Met
1040                1045                1050

taa aaa tgt ccc ccc cgc tcc gtc ccc caa aca tgt tgt aca ttt 3249
Lys Cys Pro Pro Arg Ser Val Pro Gln Thr Cys Cys Thr Phe
1055                1060                1065

cac cat ggc ccc ctc atc ata gca ata aca ttc cca ctg cca ggg 3294
His His Gly Pro Leu Ile Ile Ala Ile Thr Phe Pro Leu Pro Gly
    1070                1075                1080

gtt ctt gag cca gcc agg ccc tgc cag tgg gga agg agg cca agc 3339
Val Leu Glu Pro Ala Arg Pro Cys Gln Trp Gly Arg Arg Pro Ser
    1085                1090                1095

agt gcc tgc cta tga aat ttc aac ttt tcc ttt cat acg tct tta 3384
Ser Ala Cys Leu Asn Phe Asn Phe Ser Phe His Thr Ser Leu
    1100                1105                1110

tta ccc aag tct tct ccc gtc cat tcc agt caa atc tgg gct cac 3429
Leu Pro Lys Ser Ser Pro Val His Ser Ser Gln Ile Trp Ala His
        1115                1120                1125

tca ccc cag cga gct ctc aaa tcc ctc tcc aac tgc cta agg ccc 3474
Ser Pro Gln Arg Ala Leu Lys Ser Leu Ser Asn Cys Leu Arg Pro
        1130                1135                1140

ttt gtg taa ggt  gtc tta ata ctg tcc  ttt ttt ttt ttt taa cag        3519
Phe Val     Gly  Val Leu Ile Leu Ser  Phe Phe Phe Phe     Gln
            1145                 1150                     1155

tgt ttt gta gat ttc  aga tga cta tgc aga ggc  ctg ggg gac ccc        3564
Cys Phe Val Asp Phe  Arg     Leu Cys Arg Gly  Leu Gly Asp Pro
                1160                     1165

tgg  ctc tgg gcc ggg cct  ggg gct ccg aaa ttc  caa ggc cca gac       3609
Trp  Leu Trp Ala Gly Pro  Gly Ala Pro Lys Phe  Gln Gly Pro Asp
1170                 1175                 1180

ttg  cgg ggg gtg ggg ggg  tat cca gaa ttg gtt  gta aat act ttg       3654
Leu  Arg Gly Val Gly Gly  Tyr Pro Glu Leu Val  Val Asn Thr Leu
1185                 1190                 1195

cat  att gtc tga tta aac aca  aac aga cct cag aaa  aaa aaa aaa       3699
His  Ile Val     Leu Asn Thr  Asn Arg Pro Gln Lys  Lys Lys Lys
1200                     1205                 1210

aaa aaa  aaa aaa                                                     3711
Lys Lys  Lys Lys
    1215

<210>  5

<211>  1626

<212>  DNA

<213>  Murinae gen. sp.


<220>

<221>  exon

<222>  (1)..(1626)

<223>


<400>  5
atg ggc agc aac aag agc aag ccc aag gac gcc agc cag cgg cgc cgc       48
Met Gly Ser Asn Lys Ser Lys Pro Lys Asp Ala Ser Gln Arg Arg Arg
1               5                   10                  15

agc ctg gag ccc tcg gaa aac gtg cac ggg gca ggg ggc gcc ttc ccg       96
Ser Leu Glu Pro Ser Glu Asn Val His Gly Ala Gly Gly Ala Phe Pro
            20                  25                  30

gcc tca cag aca ccg agc aag ccc gcc tcc gcc gac ggc cac cgc ggg      144
Ala Ser Gln Thr Pro Ser Lys Pro Ala Ser Ala Asp Gly His Arg Gly
        35                  40                  45

ccc agc gcc gcc ttc gtg ccg ccc gcg gcc gag ccc aag ctc ttc gga      192
Pro Ser Ala Ala Phe Val Pro Pro Ala Ala Glu Pro Lys Leu Phe Gly
    50                  55                  60

ggc ttc aac tcc tcg gac acc gtc acc tcc ccg cag agg gcg ggc gct      240
Gly Phe Asn Ser Ser Asp Thr Val Thr Ser Pro Gln Arg Ala Gly Ala
65                  70                  75                  80

ctg gca ggt ggg gtg acc acc ttt gtg gcc ctc tat gac tat gag tca      288

Leu Ala Gly Gly Val Thr Thr Phe Val Ala Leu Tyr Asp Tyr Glu Ser
                85                  90                  95

cgg aca gag act gac ctg tcc ttc aag aaa ggg gag cgg ctg cag att      336
Arg Thr Glu Thr Asp Leu Ser Phe Lys Lys Gly Glu Arg Leu Gln Ile
            100                 105                 110

gtc aat aac acg agg aag gtg gat gtc aga gag gga gac tgg tgg ctg      384
Val Asn Asn Thr Arg Lys Val Asp Val Arg Glu Gly Asp Trp Trp Leu
        115                 120                 125

gca cac tcg ctg agc acg gga cag acc ggt tac atc ccc agc aac tat      432
Ala His Ser Leu Ser Thr Gly Gln Thr Gly Tyr Ile Pro Ser Asn Tyr
    130                 135                 140

gtg gcg ccc tcc gac tcc atc cag gct gag gag tgg tac ttt ggc aag      480
Val Ala Pro Ser Asp Ser Ile Gln Ala Glu Glu Trp Tyr Phe Gly Lys
145                 150                 155                 160

atc act aga cgg gaa tca gag cgg ctg ctg ctc aac gcc gag aac ccg      528
Ile Thr Arg Arg Glu Ser Glu Arg Leu Leu Leu Asn Ala Glu Asn Pro
                165                 170                 175

aga ggg acc ttc ctc gtg agg gag agt gag acc aca aaa ggt gcc tac      576
Arg Gly Thr Phe Leu Val Arg Glu Ser Glu Thr Thr Lys Gly Ala Tyr
            180                 185                 190

tgc ctc tct gta tcc gac ttc gac aat gcc aag ggc cta aat gtg aaa      624
Cys Leu Ser Val Ser Asp Phe Asp Asn Ala Lys Gly Leu Asn Val Lys
        195                 200                 205

cac tac aag atc cgc aag ctg gac agc ggc ggt ttc tac atc acc tcc      672
His Tyr Lys Ile Arg Lys Leu Asp Ser Gly Gly Phe Tyr Ile Thr Ser
    210                 215                 220

cgc acc cag ttc aac agc ctg cag cag ctc gtg gct tac tac tcc aaa      720
Arg Thr Gln Phe Asn Ser Leu Gln Gln Leu Val Ala Tyr Tyr Ser Lys
225                 230                 235                 240

cat gct gat ggc ctg tgt cac cgc ctc act acc gta tgt ccc aca tcc      768
His Ala Asp Gly Leu Cys His Arg Leu Thr Thr Val Cys Pro Thr Ser
                245                 250                 255

aag cct cag acc cag gga ttg gcc aag gat gcg tgg gag atc ccc cgg      816
Lys Pro Gln Thr Gln Gly Leu Ala Lys Asp Ala Trp Glu Ile Pro Arg
            260                 265                 270

gag tcc ctg cgg ctg gag gtc aag ctg ggc cag ggt tgc ttc gga gag      864
Glu Ser Leu Arg Leu Glu Val Lys Leu Gly Gln Gly Cys Phe Gly Glu
        275                 280                 285

gtg tgg atg ggg acc tgg aac ggc acc acg agg gtt gcc atc aaa act      912
Val Trp Met Gly Thr Trp Asn Gly Thr Thr Arg Val Ala Ile Lys Thr
    290                 295                 300

ctg aag cca ggc acc atg tcc cca gag gcc ttc ctg cag gag gcc caa      960
Leu Lys Pro Gly Thr Met Ser Pro Glu Ala Phe Leu Gln Glu Ala Gln
305                 310                 315                 320

gtc atg aag aaa ctg agg cac gag aaa ctg gtg cag ctg tat gct gtg     1008
Val Met Lys Lys Leu Arg His Glu Lys Leu Val Gln Leu Tyr Ala Val
                325                 330                 335

gtg tcg gaa gaa ccc att tac att gtg aca gag tac atg aac aag ggg     1056
Val Ser Glu Glu Pro Ile Tyr Ile Val Thr Glu Tyr Met Asn Lys Gly
            340                 345                 350

agt ctg ctg gac ttt ctc aag ggg gaa acg ggc aaa tat ttg cgg cta     1104

Ser Leu Leu Asp Phe Leu Lys Gly Glu Thr Gly Lys Tyr Leu Arg Leu
        355                 360                 365

ccc cag ctg gtg gac atg tct gct cag atc gct tca ggc atg gcc tat      1152
Pro Gln Leu Val Asp Met Ser Ala Gln Ile Ala Ser Gly Met Ala Tyr
    370                 375                 380

gtg gag cgg atg aac tat gtg cac cgg gac ctt cga gcc gcc aat atc      1200
Val Glu Arg Met Asn Tyr Val His Arg Asp Leu Arg Ala Ala Asn Ile
385                 390                 395                 400

cta gta ggg gag aac ctg gtg tgc aaa gtg gcc gac ttt ggg ttg gcc      1248
Leu Val Gly Glu Asn Leu Val Cys Lys Val Ala Asp Phe Gly Leu Ala
                405                 410                 415

cgg ctc ata gaa gac aac gaa tac aca gcc cgg caa ggt gcc aaa ttc      1296
Arg Leu Ile Glu Asp Asn Glu Tyr Thr Ala Arg Gln Gly Ala Lys Phe
            420                 425                 430

ccc atc aag tgg acc gcc cct gaa gct gct ctg tac ggc agg ttc acc      1344
Pro Ile Lys Trp Thr Ala Pro Glu Ala Ala Leu Tyr Gly Arg Phe Thr
        435                 440                 445

atc aag tcg gat gtg tgg tcc ttt ggg att ctg ctg acc gag ctc acc      1392
Ile Lys Ser Asp Val Trp Ser Phe Gly Ile Leu Leu Thr Glu Leu Thr
    450                 455                 460

act aag gga aga gtg ccc tat cct ggg atg gtg aac cgt gag gtt ctg      1440
Thr Lys Gly Arg Val Pro Tyr Pro Gly Met Val Asn Arg Glu Val Leu
465                 470                 475                 480

gac cag gtg gag cgg ggc tac cgg atg cct tgt ccc ccc gag tgc ccc      1488
Asp Gln Val Glu Arg Gly Tyr Arg Met Pro Cys Pro Pro Glu Cys Pro
                485                 490                 495

gag tcc ctg cat gac ctt atg tgc cag tgc tgg cgg aag gag ccc gag      1536
Glu Ser Leu His Asp Leu Met Cys Gln Cys Trp Arg Lys Glu Pro Glu
            500                 505                 510

gag cgg ccc acc ttc gag tac ctg cag gcc ttc ctg gaa gac tac ttt      1584
Glu Arg Pro Thr Phe Glu Tyr Leu Gln Ala Phe Leu Glu Asp Tyr Phe
        515                 520                 525

acg tcc act gag cca cag tac cag ccc ggg gag aac cta tag              1626
Thr Ser Thr Glu Pro Gln Tyr Gln Pro Gly Glu Asn Leu
    530                 535                 540

## Claims

1. Method for identifying a therapeutic compound for the treatment of Alzheimer's disease comprising the steps
a) providing a Src protein and
b) determining the inhibitory effect of a compound on the Src activity.

2. Method of identifying a therapeutic compound for the treatment of Alzheimer' s disease comprising the steps
a) providing a sequence which regulates Src expression and
b) determining if a compound inhibits the expression of Src protein.

3. Method according to claim 1 or 2, wherein Src is human Src or mouse Src.

4. Method according to one of the forgoing claims, wherein Src has the sequence SEQ ID NO. 1, SEQ ID NO. 2 or SEQ ID NO. 3.

5. Method according to claim 1, wherein Src is expressed in a mammalian cell.

6. Method according to claim 2, wherein the regulating sequence is the Src promotor.

7. Method of preparing a pharmaceutical for the treatment of Alzheimer's disease comprising the steps
a) identification of a therapeutic compound by using one of the methods claimed in claims 1 to 6;
b) optimisation of the identified compound; and
c) formulation of the optimised compound.

8. Use of a compound identified by a method according to claims 1 to 6 for the preparation of a pharmaceutical for treating Alzheimer's disease.

9. PP2 for use as pharmaceutical.

10. Use of PP2 for the preparation of a pharmaceutical for the treatment of Alzheimer's disease.
